# EUROPÄISCHE PATENTSCHRIFT

(11) **EP 1 451 195 B1**
(45) Veröffentlichungstag und Bekanntmachung des Hinweises auf die Patenterteilung: **20.07.2011**
(21) Anmeldenummer: 02803777.8
(22) Anmeldetag: 18.11.2002
(51) Int. Cl.: C07D 513/04, C07D 307/60, C07D 493/10, C07D 495/10, C07D 207/42, C07D 277/34, A01N 43/08, A01N 43/10, A01N 43/36

(54) **4-KETOLACTAMES ET KETOLACTONES SUBSTITUES EN 3 ET BIPHENYL-SUBSTITUES EN 3 ET LEUR UTILISATION EN TANT QUE PESTICIDES**
3-BIPHENYL-SUBSTITUTED-3-SUBSTITUTED-4-KETOLACTAMS AND KETOLACTONES AND THEIR UTILIZATION AS PESTICIDE
4-CETOLACTAMES ET CETOLACTONES BIPHENYL-SUBSTITUES EN 3 ET SUBSTITUES EN 3 ET LEUR UTILISATION EN TANT QUE PESTICIDES

(30) Priorität: 29.11.2001 DE 10158560
(43) Veröffentlichungstag der Anmeldung: 01.09.2004
(73) Patentinhaber: Bayer CropScience AG, 40789 Monheim (DE)
(72) Erfinder: FISCHER, Reiner, 40789 Monheim (DE); ULLMANN, Astrid, 50677 Köln (DE); BRETSCHNEIDER, Thomas, 53797 Lohmar (DE); DREWES, Mark, Wilhelm, 40764 Langenfeld (DE); ERDELEN, Christoph, deceased, (DE); FEUCHT, Dieter, 65779 Kelkheim (DE); RECKMANN, Udo, 50823 Köln (DE); KUCK, Karl-Heinz, 40764 Langenfeld (DE); WACHENDORFF-NEUMANN, Ulrike, 56566 Neuwied (DE)
(86) Internationale Anmeldenummer: PCT/EP2002/012881
(87) Internationale Veröffentlichungsnummer: WO 2003/045957

(56) Entgegenhaltungen:
- WO-A-96/35664
- JP-A- 2000 086 628

## Beschreibung

Die Erfindung betrifft neue 3-Biphenylsubstituierte-3-substituierte-4-ketolaktame und -lakton, Verfahren und Zwischenprodukte zu ihrer Herstellung und ihre Verwendung als Schädlingsbekämpfungsmittel, Mikrobizide und/oder Herbizide.

Es ist bereits bekannt geworden, dass bestimmte phenylsubstituierte 3-Halogen-4-ketolaktame (JP-A-10-258 555) und phenylsubstituierte 3-Halogen-4-ketolaktone (JP-A-10-258 555) als Akarizide, Insektizide und/oder Herbizide wirksam sind.

Die Wirksamkeit und Wirkungsbreite dieser Verbindungen ist jedoch insbesondere bei niedrigen Aufwandmengen und Konzentrationen nicht immer völlig zufriedenstellend. Weiterhin ist die Pflanzenverträglichkeit dieser Verbindungen in den Kulturpflanzen nicht immer ausreichend.

Es wurden nun neue Verbindungen der Formel (I) gefunden, 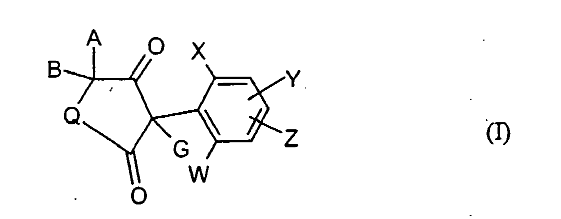 in welcher
- Q: für Sauerstoff, Schwefel oder die Gruppe N-D steht,
- W: für Wasserstoff, Halogen, C₁-C₆-Alkyl oder C₁-C₆-Alkoxy steht,
- X: für Halogen, C₁-C₆-Alkyl, C₁-C₆-Halogenalkyl, C₁-C₆-Akoxy, C₁- C₆-Halogenalkoxy, Cyano oder gegebenenfalls durch Halogen, C₁- C₆-Alkyl, C₁-C₆-Alkoxy, C₁-C₄-Halogenalkyl, C₁-C₄-Halogen- alkoxy, Nitro oder Cyano substituiertes Phenyl steht, 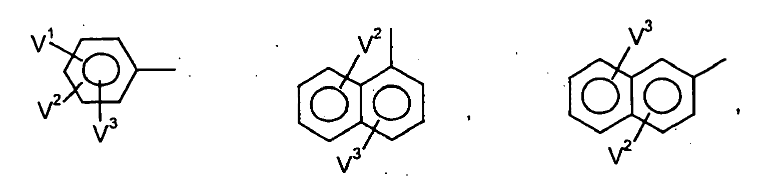 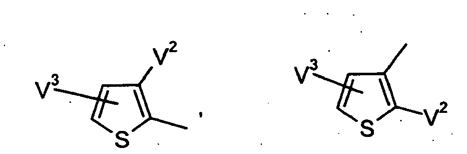 steht,
- V¹: für Wasserstoff, Halogen, C₁-C₁₂-Alkyl, C₁-C₆-Alkoxy, C₁-C₆- Alkylthio, C₁-C₆-Alkylsulfinyl, C₁-C₆-Alkylsulfonyl, C₁-C₄-Halogen- alkyl, C₁-C₄-Halogenalkoxy, Nitro, Cyano oder gegebenenfalls einfach bis zweifach durch Halogen, C₁-C₆-Akyl, C₁-C₆-Alkoxy, C₁- C₄-Halogenalkyl, C₁-C₄-Halogenalkoxy, Nitro oder Cyano substituiertes Phenyl steht,
- V² und V³: unabhängig voneinander für Wasserstoff, Halogen, C₁-C₆-Akyl, C₁-C₆-Alkoxy, C₁-C₄-Halogenakyl oder C₁-C₄-Halogenalkoxy stehen,
- Z: für Wasserstoff, Halogen, C₁-C₆-Alkyl, C₁-C₆-Halogenalkyl, C₁-C₆- Alkoxy, C₁-C₆-Halogenalkoxy, Nitro oder Cyano steht,
- A: für jeweils gegebenenfalls durch Halogen substituiertes C₁-C₁₂-Alkyl, C₃-C₈-Alkenyl, C₁-C₁₀-Alkoxy-C₁-C₈-alkyl, Poly-C₁-C₈-alkoxy-C₁- C₈-alkyl, C₁-C₁₀-Alkylthio-C₁-C₆-alkyl, gegebenenfalls durch Halo- gen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Phenyl-C₁-C₆-alkyl oder für Wasserstoff steht, wenn im Falle der Gruppe (I-1) D ungleich Wasserstoff ist,
- B: für Wasserstoff, C₁-C₁₂-Alkyl oder C₁-C₈-Alkoxy-C₁-C₆-alkyl steht oder
- A, B: und das Kohlenstoffatom an das sie gebunden sind, für gesättigtes C₃- C₁₀-Cycloakyl oder ungesättigtes C₅-C₁₀-Cycloalkyl stehen, worin gegebenenfalls ein Ringglied durch Sauerstoff oder Schwefel ersetzt ist und welche gegebenenfalls einfach oder zweifach durch C₁-C₈- Alkyl, C₃-C₁₀-Cycloalkyl, C₁-C₆-Halogenalkyl, C₁-C₈₋Alkoxy, C₁- C₈-Alkylthio, Halogen oder Phenyl substituiert sind oder
- A, B: und das Kohlenstoffatom, an das sie gebunden sind, für C₃-C₆- Cycloalkyl stehen, welches durch eine gegebenenfalls ein oder zwei nicht direkt benachbarte Sauerstoff- und/oder Schwefelatome enthaltende Alkylendiyl-, oder durch eine Alkylendioxyl- oder durch eine Alkylendithioyl-Gruppe substituiert ist, die mit dem Kohlen- stoffatom, an das sie gebunden ist, einen weiteren fünf- bis acht- gliedrigen Ring bildet oder
- A, B: und das Kohlenstoffatom, an das sie gebunden sind, für C₃-C₈- Cycloalkyl oder C₅-C₈-Cycloalkenyl stehen, in welchen zwei Sub- stituenten gemeinsam mit den Kohlenstoffatomen, an die sie gebunden sind, für jeweils gegebenenfalls durch C₁-C₆-Alkyl, C₁-C₆-Akoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
- D: für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂- C₈-alkyl, Poly-C₁-C₈-alkoxy-C₂-C₈-alkyl, C₁-C₁₀-Alkylthio-C₂-C₈- alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder gegebenenfalls durch Halogen, C₁-C₆-Alkyl, C₁-C₆- Halogenalkyl, C₁-C₆-Alkoxy, C₁-C₆-Halogenalkoxy, Cyano oder Nitro substituiertes Phenyl steht,
- A und D: gemeinsam für jeweils gegebenenfalls substituiertes C₃-C₆- Alkandiyl oder C₃-C₆-Akendiyl stehen, worin gegebenenfalls eine Methylengruppe durch Sauerstoff oder Schwefel ersetzt ist und wobei als Substituenten jeweils in Frage kommen: C₁-C₆-Alkyl, C₁-C₆-Alkoxy oder eine weitere C₃-C₆-Alkandiyl- gruppierung,
- G: für Chlor, Brom oder Nitro steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen für Q ergeben sich folgende hauptsächliche Strukturen (I-1) bis (I-3): 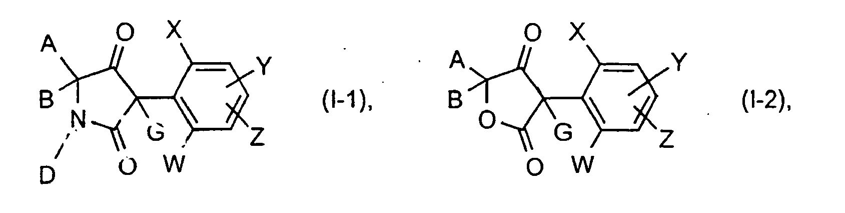 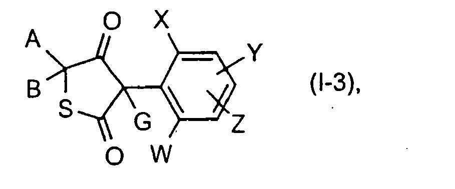 worin
A, B, D, G, W, X, Y und Z die oben angegebene Bedeutung haben.

### A) Weiterhin wurde gefunden, dass man Verbindungen der Formeln (I-1) bis (I-3) erhält

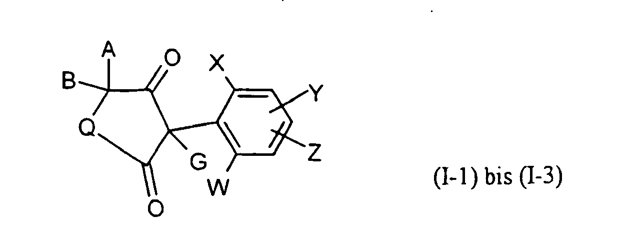

in welcher A, B, Q, W, X, Y und Z, die oben angegebene Bedeutung haben
und
- G: für Halogen, bevorzugt für Chlor und Brom steht,
wenn man Verbindungen der Formeln (II-1) bis (II-3) 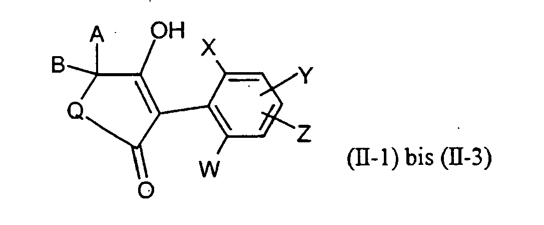 in welcher A, B, Q, W, X, Y und Z die oben angegebene Bedeutung haben
mit Halogenierungsmittel in Gegenwart eines Lösungsmittels und gegebenenfalls in Gegenwart eines Radikalstarters umsetzt.

### B) Weiterhin erhält man Verbindungen der Formeln (I-1) bis (I-3)

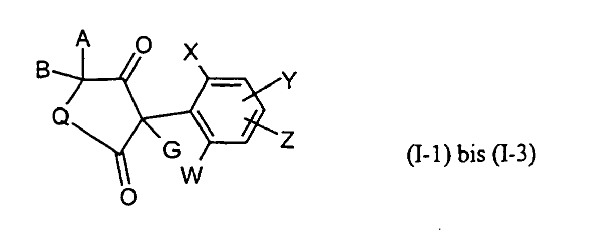

in welcher A, B, Q, W, X, Y und Z die oben angegebene Bedeutung haben
und
- G: für Nitro steht,
wenn man Verbindungen der Formeln (II-1) bis (II-3) 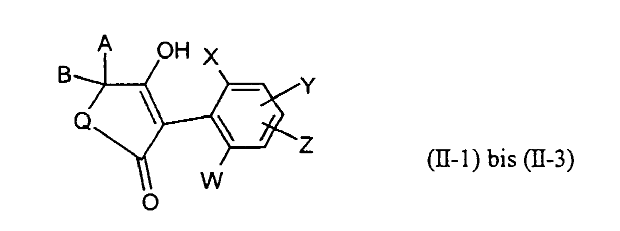 in welcher
A, B, Q, W, X, Y und Z die oben angegebene Bedeutung haben
mit Nitrierungsreagenzien wie z.B. rauchende Salpetersäure in Gegenwart eines Lösungsmittels umsetzt.

Die für die Verfahren A und B benötigten Verbindungen der Formeln (II-1) bis (II-3) 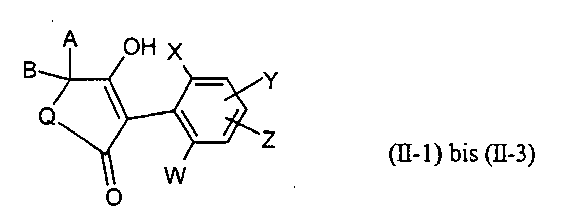 in welcher
A, B, Q, W, X, Y und Z die oben angegebene Bedeutung haben,
sind bekannte Verbindungen (WO 99/43649, WO 99/48869, WO 99/55673) oder lassen sich nach den dort beschriebenen Verfahren synthetisieren.

Als Halogenierungsmittel kommen für das Verfahren A beispielsweise Sulfurylchlorid, Sulfurylbromid, Thionylchlorid, Thionylbromid, Imide wie z.B. N-Bromsuccinimid oder N-Chlorsuccinimid, Chlorsulfonsäure aber auch Hypochlorite wie z.B. tert.-Butylhypochlorit in Frage.

Als Nitrierungsreagenzien kommen für das Verfahren B rauchende Salpetersäure aber auch "Nitriersäuremischungen" in Frage.

Weiterhin wurde gefunden, dass die neuen Verbindungen der Formel (I) eine sehr gute Wirksamkeit als Schädlingsbekämpfungsmittel, vorzugsweise als Insektizide, Akarizide und/oder Fungizide und/oder Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:

In den bevorzugten Restedefinitionen steht Halogen für Fluor, Chlor, Brom und Iod, insbesondere für Fluor, Chlor und Brom.
- Q: steht besonders bevorzugt für Sauerstoff, Schwefel oder die Gruppe N-D,
- W: steht besonders bevorzugt für Wasserstoff, Chlor, Brom oder C₁-C₄-Alkyl,
- X: steht besonders bevorzugt für Fluor, Chlor, Brom, C₁-C₄-Alkyl, C₁-C₄-Alk- oxy, C₁-C₄-Halogenalkyl, C₁-C₄-Halogenalkoxy oder Cyano,
- Y: steht besonders bevorzugt für den Rest

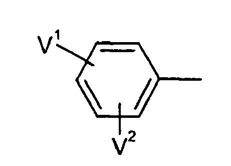

- V¹: steht besonders bevorzugt für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro oder Cyano, gegebenenfalls einfach oder zweifach durch Fluor, Chlor, Brom, C₁- C₄-Alkyl, C₁-C₄-Alkoxy, C₁-C₂-Halogenalkyl, C₁-C₂-Halogenalkoxy, Nitro oder Cyano substituiertes Phenyl,
- V²: steht besonders bevorzugt für Wasserstoff, Fluor, Chlor, Brom, C₁-C₄-Alkyl, C₁-C₄-Alkoxy, C₁-C₂-Halogenalkyl oder C₁-C₂-Halogenalkoxy,
- Z: steht besonders bevorzugt für Wasserstoff, Fluor, Chlor, Brom, C₁-C₄-Alkyl, C₁-C₂-Halogenalkyl, C₁-C₄-Alkoxy oder C₁-C₂-Halogenalkoxy,
- A: steht besonders bevorzugt für jeweils gegebenenfalls einfach bis fünffach durch Fluor oder Chlor substituiertes C₁-C₁₀-Alkyl, C₁-C₈-Alkoxy-C₁-C₆- alkyl, gegebenenfalls einfach bis zweifach durch Fluor, Chlor, C₁-C₄-Alkyl oder C₁-C₄-Alkoxy substituiertes C₃-C₇-Cycloalkyl, in welchem gegebenenfalls ein Ringglied durch Sauerstoff oder Schwefel ersetzt ist oder jeweils gegebenenfalls einfach bis zweifach durch Fluor, Chlor, Brom, C₁- C₄-Alkyl, C₁-C₄-Halogenalkyl, C₁-C₄-Alkoxy oder C₁-C₄-Halogenalkoxy substituiertes Phenyl oder Phenyl-C₁-C₄-alkyl oder für Wasserstoff, wenn im Falle der Gruppe (I-1) D ungleich Wasserstoff ist,
- B: steht besonders bevorzugt für Wasserstoff oder C₁-C₆-Alkyl oder
- A, B: und das Kohlenstoffatom an das sie gebunden sind, stehen besonders bevor- zugt für gesättigtes C₃-C₈-Cycloalkyl, worin gegebenenfalls ein Ringglied durch Sauerstoff oder Schwefel ersetzt ist und welches gegebenenfalls einfach bis zweifach durch C₁-C₆-Alkyl, C₅-C₆-Cycloalkyl, C₁-C₃-Halogenalkyl, C₁-C₆-Alkoxy, Fluor, Chlor oder Phenyl substituiert ist, mit der Maßgabe, dass D in der Gruppe (I-1) für Wasserstoff oder C₁-C₄-Alkyl steht,
- D: steht besonders bevorzugt für Wasserstoff, für jeweils gegebenenfalls einfach bis fünffach durch Fluor oder Chlor substituiertes C₁-C₁₀-Alkyl, C₃-C₆- Alkenyl, C₁-C₄-Alkoxy-C₂-C₄-alkyl oder C₁-C₄-Alkylthio-C₂-C₄-alkyl, für gegebenenfalls einfach bis zweifach durch Fluor, Chlor, C₁-C₄-Alkyl, C₁- C₄-Alkoxy oder C₁-C₂-Halogenalkyl substituiertes C₃-C₇-Cycloalkyl, in welchem gegebenenfalls eine Methylengruppe durch Sauerstoff oder Schwefel ersetzt ist oder für gegebenenfalls einfach bis zweifach durch Fluor, Chlor, Brom, C₁-C₄-Alkyl, C₁-C₄-Halogenalkyl, C₁-C₄-Alkoxy oder C₁- C₄-Halogenalkoxy substituiertes Phenyl, oder
- A und D: stehen gemeinsam besonders bevorzugt für gegebenenfalls substituiertes C₃-C₅-Alkandiyl, in welchem eine Methylengruppe durch Sauerstoff oder Schwefel ersetzt sein kann, wobei als Substituenten C₁-C₄-Alkyl in Frage kommen,
- G: steht besonders bevorzugt für Chlor, Brom oder Nitro.

In den als besonders bevorzugt genannten Restedetinitionen steht Halogen für Fluor, Chlor, Brom und Iod, insbesondere für Fluor, Chlor und Brom.
- Q: steht ganz besonders bevorzugt für Sauerstoff, Schwefel oder die Gruppe N- D,
- W: steht ganz besonders bevorzugt für Wasserstoff, Chlor, Methyl oder Ethyl,
- X: steht ganz besonders bevorzugt für Chlor, Methyl, Ethyl, n-Propyl, iso- Propyl, Methoxy, Ethoxy, n-Propoxy, iso-Propoxy, Trifluormethyl, Difluor- methoxy, Trifluormethoxy oder Cyano,
- Y: steht ganz besonders bevorzugt für den Rest

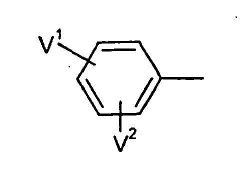

- V¹: steht ganz besonders bevorzugt für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro oder Cyano,
- V²: steht ganz besonders bevorzugt für Wasserstoff, Fluor, Chlor, Methyl, Ethyl, n-Propyl, iso-Propyl, Methoxy, Ethoxy, Trifluormethyl oder Trifluormethoxy,
- Z: steht ganz besonders bevorzugt für Wasserstoff, Fluor, Chlor, Methyl, Ethyl, n-Propyl oder Methoxy,
- A: steht ganz besonders bevorzugt für C₁-C₆-Alkyl oder C₁-C₂-Alkoxy-C₁-C₂- alkyl, C₃-C₆-Cycloalkyl, in welchem gegebenenfalls ein Ringglied durch Sauerstoff oder Schwefel ersetzt ist oder für Wasserstoff, wenn im Falle der Gruppe (I-1) D ungleich Wasserstoff ist.
- B: steht ganz besonders bevorzugt für Wasserstoff, Methyl, Ethyl oder
- A, B: und das Kohlenstoffatom an das sie gebunden sind, stehen ganz besonders bevorzugt für gesättigtes C₃-C₈-Cycloalkyl, in welchem gegebenenfalls ein Ringglied durch Sauerstoff oder Schwefel ersetzt ist und welches gegebenen- falls einfach durch Methyl, Ethyl, n-Propyl, Isopropyl, n-Butyl, Isobutyl, tert.- Butyl, Trifluormethyl, Methoxy, Ethoxy, n-Propoxy, iso-Propoxy, n-Butoxy oder iso-Butoxy substituiert ist, mit der Maßgabe, dass D in der Gruppe (I-1) für Wasserstoff oder C₁-C₃-Alkyl insbesondere aber für Wasserstoff steht,
- D: steht ganz besonders bevorzugt für Wasserstoff, Methyl, Ethyl, n-Propyl, Isopropyl, Cyclopropyl, Cyclopentyl oder Cyclohexyl, insbesondere bevor- zugt für Wasserstoff,
- A, D: stehen gemeinsam ganz besonders bevorzugt für gegebenenfalls substituiertes C₃-C₄-Alkandiyl, worin gegebenenfalls ein Kohlenstoffatom durch Sauerstoff oder Schwefel ersetzt ist,
- G: steht ganz besonders bevorzugt für Chlor oder Nitro, insbesondere bevorzugt für Chlor.

Insbesondere bevorzugt sind Verbindungen der Formel (I-1) in welcher
- W: insbesonders bevorzugt für Wasserstoff, Chlor oder Methyl steht,
- X: insbesonders bevorzugt für Chlor, Methyl oder Ethyl steht,
- Y: insbesonders bevorzugt für den Rest 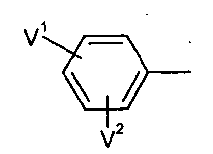 steht,
V¹ insbesonders bevorzugt für Chlor oder Methyl steht,
V² insbesonders bevorzugt für Wasserstoff oder Chlor steht, Z insbesonders bevorzugt für Wasserstoff oder Methyl steht,
A insbesonders bevorzugt für Methyl, Ethyl, Propyl, Isopropyl, n-Butyl, Isobutyl, s-Butyl, tert.-Butyl, Cyclopropyl, Cyclopentyl oder Cyclohexyl steht (hervorgehoben für Methyl oder Isopropyl steht),
B insbesonders bevorzugt für Methyl steht,
A,B und das Kohlenstoffatom, an das sie gebunden sind, insbesonders bevorzugt für gesättigtes C₃-C₈-Cycloalkyl steht, in welchem gegebenenfalls ein Ring- glied durch Sauerstoff oder Schwefel ersetzt ist und welches gegebenenfalls einfach durch Methyl oder im Falle von C₆-Cycloalkyl auch durch Ethyl, n- Propyl, Isopropyl, n-Butyl, Isobutyl, tert.-Butyl, Trifluormethyl, Methoxy, Ethoxy, n-Propoxy, iso-Propoxy, n-Butoxy oder iso-Butoxy substituiert ist, (hervorgehoben für C₃-, C₅- oder C₆-Cycloalkyl, worin im Falle von C₆- Cycloalkyl gegebenenfalls ein Ringatom durch Sauerstoff ersetzt ist und welches gegebenenfalls einfach durch Methyl, Ethyl, n-Propyl, Isopropyl, Methoxy oder Ethoxy substituiert ist),
D insbesonders bevorzugt für Wasserstoff steht,
G insbesonders bevorzugt für Chlor oder Nitro steht (hervorgehoben für Chlor steht).

Insbesonders bevorzugt sind Verbindungen der Formel (I-2) in welcher
- W: insbesonders bevorzugt für Wasserstoff, Chlor oder Methyl steht, (hervorgehoben für Wasserstoff oder Methyl),
- X: insbesonders bevorzugt für Chlor, Methyl oder Ethyl steht, (hervorgehoben für Methyl oder Ethyl),
- Y: insbesonders bevorzugt für den Rest 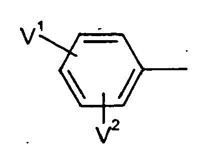
steht,
(hervorgehoben für 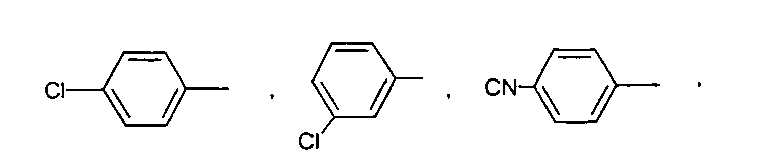 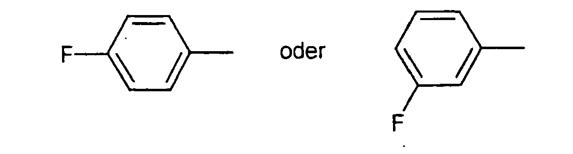
- V¹: insbesonders bevorzugt für Chlor, Fluor, Cyano oder Methyl steht,
- V²: insbesonders bevorzugt für Wasserstoff oder Chlor steht, Z insbesonders bevorzugt für Wasserstoff oder Methyl steht,
- A: insbesonders bevorzugt für Methyl, Ethyl; Propyl, Isopropyl, n-Butyl, Isobutyl, s-Butyl, tert.-Butyl, Cyclopropyl, Cyclopentyl oder Cyclohexyl steht, (hervorgehoben für Methyl oder Isopropyl steht),
- B: insbesonders bevorzugt für Methyl steht oder
- A, B: und das Kohlenstoffatom an das sie gebunden sind, insbesonders bevorzugt für gesättigtes C₃-C₈-Cycloalkyl steht, in welchem gegebenenfalls ein Ringglied durch Sauerstoff oder Schwefel ersetzt ist und welches gegebenen- falls einfach durch Methyl oder im Falle von C₆-Cycloalkyl auch durch Ethyl, n-Propyl, Isopropyl, n-Butyl, Isobutyl, tert.-Butyl, Trifluormethyl, Methoxy, Ethoxy, n-Propoxy, iso-Propoxy, n-Butoxy oder iso-Butoxy substituiert ist; (hervorgehoben für C₅- oder C₆-Cycloalkyl, worin im Falle von C₆-Cycloalkyl gegebenenfalls ein Ringatom durch Sauerstoff ersetzt ist und welches gegebenenfalls einfach durch Methyl, Trifluormethyl, Methoxy oder Ethoxy substituiert ist),
- G: insbesonders bevorzugt für Chlor steht.

Insbesonders bevorzugt sind Verbindungen der Formel (1-3) in welcher
- W: insbesonders bevorzugt für Wasserstoff steht,
- X: insbesonders bevorzugt für Chlor oder Methyl steht, (hervorgehoben für Methyl steht),
- Y: insbesonders bevorzugt für den Rest 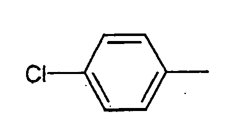 steht ,

- Z: insbesondere bevorzugt für Wasserstoff oder Methyl steht,
- A, B: und das Kohlenstoffatom, an das sie gebunden sind, insbesonders bevorzugt für gesättigtes C₅-C₆-Cycloalkyl steht, in welchem gegebenenfalls ein Ringglied durch Sauerstoff ersetzt ist und welches gegebenenfalls durch Methyl oder Methoxy substituiert ist, (hervorgehoben für C₆-Cycloakyl steht),
- G: insbesonders bevorzugt für Chlor steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend. Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1) genannt:

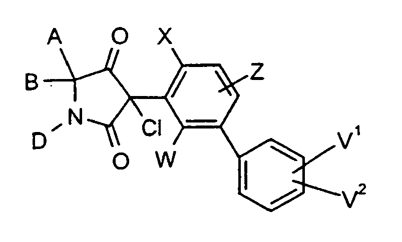

**Tabelle 1: W=H; X=CH₃, V¹=H, V²=H, Z=H**

| A | B | D |
|---|---|---|
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| s-C₄H₉ | CH₃ | H |
| t-C₄H₉ | CH₃ | H |
| C₂H₅ | C₂H₅ | H |
| 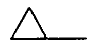 | CH₃ | H |
| 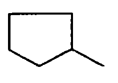 | CH₃ | H |
| 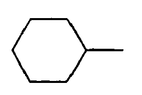 | CH₃ | H |
| -(CH2)2- | | H |
| -(CH₂)₄- | | H |
| -(CH₂)₅- | | H |
| -(CH₂)₆- | | H |
| -(CH₂)₇- | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHOi-C₃H₇-(CH₂)₂- | | H |
| -(CH₂)₂-C(CH₃)₂-(CH₂)₂- | | H |
| -CH₂-(CHCH₃)₂-(CH₂)₂- | | H |
| -(CH₂)₃- | | H |
| -(CH₂)₄- | | H |
| -CH₂-CHCH₃-CH₂- | | H |
| -CH₂-CH₂-CHCH₃- | | H |
| -CH₂-CHCH₃-CHCH₃- | | H |
| -CH₂-S-CH₂- | | H |
| -CH₂-S-(CH₂)₂- | | H |
| -(CH₂)₂-S-CH₂- | | |
| 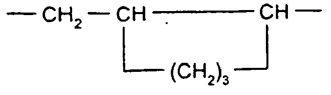 | | H |
| H | CH₃ | H |
| H | C₂H₅ | H |
| H | C₃H₇ | H |
| H | i-C₃H₇ | H |
| H | 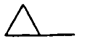 | H |
| H | 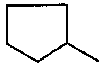 | H |
| H | 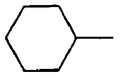 | H |
| H | CH₃ | CH₃ |
| H | C₂H₅ | CH₃ |
| H | C₃H₇ | CH₃ |
| H | i-C₃H₇ | CH₃ |
| H | 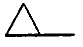 | CH₃ |
| H | 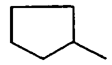 | CH₃ |
| H | 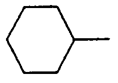 | CH₃ |
| H | CH₃ | C₂H₅ |
| H | C₂H₅ | C₂H₅ |

**Tabelle 2:** A, B und D wie in Tabelle 1 angegeben
   W = H; X = CH₃; Z=4-CH₃; V¹=H; V²=H.
**Tabelle 3:** A, B und D wie in Tabelle 1 angegeben
   W = CH₃; X = CH₃; Z = 4-CH₃; V¹ = H; V² = H.
**Tabelle 4:** A, B und D wie in Tabelle 1 angegeben
   W=H;X=CH₃; Z=H;V¹=4-Cl; V²=H.
**Tabelle 5:** A, B und D wie in Tabelle 1 angegeben
   W = H; X = CH₃; Z = 4-CH₃; V¹ = 4-Cl; V² =H.
**Tabelle 6:** A, B und D wie in Tabelle 1 angegeben
   W =CH₃; X=CH₃; Z = 4-CH₃; V¹= 4-Cl; V²= H.
**Tabelle 7:** A, B und D wie in Tabelle 1 angegeben
   W=H; X=CH₃; Z = H; V¹ = 3-Cl; V² = H.
**Tabelle 8:** A, B und D wie in Tabelle 1 angegeben
   W = H; X = CH₃; Z = 4-CH₃; V¹= 3-Cl; V² = H.
**Tabelle 9:** A, B und D wie in Tabelle 1 angegeben
   W=CH₃; X = CH₃; Z = 4-CH₃; V¹= 3-Cl; V² =H.
**Tabelle 10:** A, B und D wie in Tabelle 1 angegeben
   W = H; X = CH₃; Z =H; V¹ = 3-Cl; V² = 4-Cl.
**Tabelle 11:** A, B und D wie in Tabelle 1 angegeben
   W = H; X = CH₃; Z = 4-CH₃; V¹= 3-Cl; V² = 4-Cl.
**Tabelle 12:** A, B und D wie in Tabelle 1 angegeben
   W = CH₃; X = CH₃; Z = 4-CH₃; V¹ = 3-Cl; V² =4-Cl.
**Tabelle 13:** A, B und D wie in Tabelle 1 angegeben
   W = H; X = CH₃; Z = H; V¹ = 4-CF₃; V² = H.
**Tabelle 14:** A, B und D wie in Tabelle 1 angegeben
   W = H; X = CH₃; Z =4-CH₃; V¹= 4-CF₃; V² =H.
**Tabelle 15:** A, B und D wie in Tabelle 1 angegeben
   W = CH₃; X = CH₃; Z = 4-CH₃; V¹ = 4-CF₃; V² = H.
**Tabelle 16:** A, B und D wie in Tabelle 1 angegeben
   W = H; X = CH₃; Z = H; V¹= 4-CH₃; V² = H.
**Tabelle 17:** A, B und D wie in Tabelle 1 angegeben
   W = H; X = CH₃; Z = 4-CH₃; V¹= 4-CH₃; V² = H.
**Tabelle 18:** A, B und D wie in Tabelle 1 angegeben
   W = CH₃; X = CH₃; Z = 4-CH₃; V¹=4-CH₃; V²= H.
**Tabelle 19:** A, B und D wie in Tabelle 1 angegeben
   W = H; X = CH₃; Z = H; V¹=4-OCH₃; V² = H.
**Tabelle 20:** A, B und D wie in Tabelle 1 angegeben
   W = H; X = CH₃; Z = 4-CH₃; V¹= 4-OCH₃; V²= H.
Tabelle 21: A, B und D wie in Tabelle 1 angegeben
   W=CH₃; X = CH₃; Z=4-CH₃; V¹=4-OCH₃; V² = H.

Im einzelnen seien außer den bei den Herstellungsbeispielen genannten Verbindungen die folgenden Verbindungen der Formel (I-2) genannt:

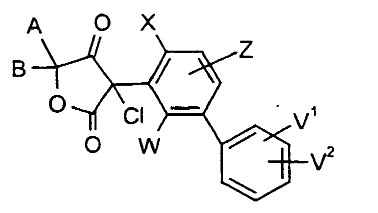

**Tabelle 22: W=H;X=CH₃, Z=H, V¹=H, V²=H.**

| **A** | **B** |
|---|---|
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| 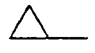 | CH₃ |
| 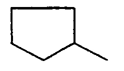 | CH₃ |
| 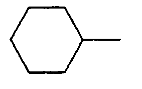 | CH₃ |
| -(CH₂)₂- | |
| -(CH₂)₄- | |
| -(CH₂)₅- | |
| -(CH₂)₆- | |
| -(CH₂)₇- | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -(CH₂)₂-CHCH₃-(CH₂)₂- | |
| (CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHOi-C₃H₇-(CH₂)₂- | |
| -(CH₂)₂-C(CH₃)₂-(CH₂)₂- | |
| -CH₂-(CHCH₃)₂-(CH₂)₂- | |
| 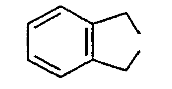 | |

**Tabelle 23:** A und B wie in Tabelle 22 angegeben
   W = H; X = CH₃; Z = 4-CH₃; V¹ = H; V² = H.
**Tabelle 24:** A und B wie in Tabelle 22 angegeben
   W = CH₃; X = CH₃; Z = 4-CH₃; V¹ = H; V² =H.
**Tabelle 25:** A und B wie in Tabelle 22 angegeben
   W = H; X = CH₃; Z = H; V¹ = 4-Cl; V² = H.
**Tabelle 26:** A und B wie in Tabelle 22 angegeben
   W = H; X = CH₃; Z = 4-CH₃; V¹ = 4-Cl; V² = H.
**Tabelle 27:** A und B wie in Tabelle 22 angegeben
   W = CH₃; X = CH₃; Z = 4-CH₃; V¹ = 4-Cl; V² = H.
**Tabelle 28:** A und B wie in Tabelle 22 angegeben
   W = H; X = CH₃; Z = H; V¹ = 3-Cl; V² = H.
**Tabelle 29:** A und B wie in Tabelle 22 angegeben
   W = H; X = CH₃; Z = 4-CH₃; V¹ = 3-Cl; V² = H.
**Tabelle 30:** A und B wie in Tabelle 22 angegeben
   W = CH₃; X = CH₃; Z = 4-CH₃; V¹ = 3-Cl; V² = H.
**Tabelle 31:** A und B wie in Tabelle 22 angegeben
   W=H,X=CH₃; Z=H; V¹=4-CF₃; V²=H.
**Tabelle 32:** A und B wie in Tabelle 22 angegeben
   W = H; X =CH₃; Z = 4-CH₃; V¹= 4-CF₃; V²= H.
**Tabelle 33:** A und B wie in Tabelle 22 angegeben
   W = CH₃; X= CH₃; Z = 4-CH₃; V¹ = 4-CF₃; V² = H.
**Tabelle 34:** A und B wie in Tabelle 22 angegeben
   W = H; X = CH₃; Z = H; V¹ = 3-Cl; V² = 4-Cl.
**Tabelle 35:** A und B wie in Tabelle 22 angegeben
   W = H; X = CH₃; Z = 4-CH₃; V¹= 3-Cl; V² =4-Cl.
**Tabelle 36:** A und B wie in Tabelle 22 angegeben
   W = CH₃; X = CH₃; Z = 4-CH₃; V¹ = 3-Cl; V¹ = 4-Cl.
**Tabelle 37:** A und B wie in Tabelle 22 angegeben
   W = H; X = CH₃; Z = H; V¹ = 4-CH₃; V² =H.
**Tabelle 38:** A und B wie in Tabelle 22 angegeben
   W = H; X = CH₃; Z = 4-CH₃; V¹ = 4-CH₃; V² = H.
**Tabelle 39:** A und B wie in Tabelle 22 angegeben
   W = CH₃; X = CH₃; Z = 4-CH₃; V¹ = 4-CH₃; V² = H.
**Tabelle 40:** A und B wie in Tabelle 22 angegeben
   W = H; X = CH₃; Z = H; V¹ = 4-OCH₃; V² = H.
**Tabelle 41:** A und B wie in Tabelle 22 angegeben
   W = H; X = CH₃; Z = 4-CH₃; V¹ = 4-OCH₃; V² = H.
**Tabelle 42:** A und B wie in Tabelle 22 angegeben
   W = CH₃; X = CH₃; Z = 4-CH₃; V¹ = 4-OCH₃; V² = H.

Verwendet man gemäß Verfahren (A) 3-[(2-Methyl-5-phenyl)-phenyl]-5,5-pentamethylen-4-hydroxy-Δ³pyrrolidin-2-on als Ausgangsstoff, so kann der Verlauf des erfindungsgemäßen Verfahrens durch folgendes Reaktionsschema wiedergegeben werden:

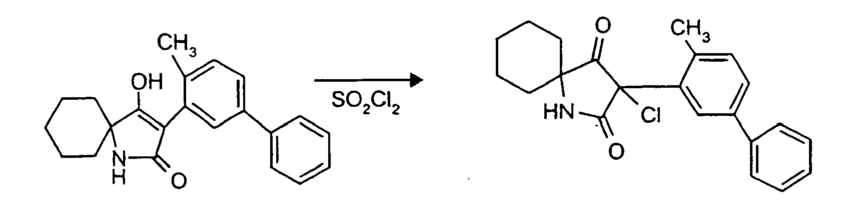

Verwendet man gemäß Verfahren (B) 3-[(2-Methyl-5-(4-chlor)-phenyl)-phenyl]-4-hydroxy-5,5-dimethyl-A³-furan-2-on, so kann der Verlauf des erfindungsgemäßen Verfahrens durch folgendes Reaktionsschema wiedergegeben werden:

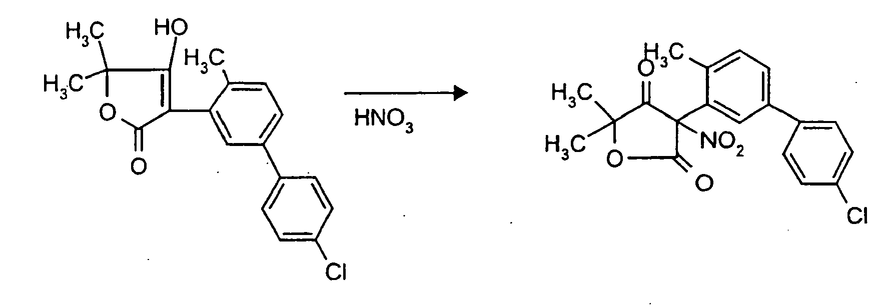

Das Verfahren (A) ist dadurch gekennzeichnet, dass man Verbindungen der Formel (II), in welcher A, B, Q, W, X, Y und Z die oben angegebenen Bedeutungen haben, in Gegenwart eines Verdünnungsmittels und eines Halogenierungsmittels und gegebenenfalls eines Radikalstarters umsetzt. Als Radikalstarter können beispielsweise Benzoylperoxid oder Azobisisobutyronitril verwendet werden.

Als Verdünnungsmittel können bei dem erfindungsgemäßen Verfahren (A) alle inerten organischen Solventien eingesetzt werden. Vorzugsweise verwendbar sind Kohlenwasserstoffe, wie Benzol, Toluol und Xylol, ferner Ether, wie Dibutylether, Tetrahydrofuran, Dioxan, Glykoldimethylether und Diglykoldimethylether, außerdem halogenierte Kohlenwasserstoffe wie Dichlormethan, Chloroform, Tetrachlorkohlenstoff, Dichlorethan, Chlorbenzol, Dichlorbenzol, aber auch Ester wie Ethylacetat.

Als Halogenierungsmittel kommen für das Verfahren (A) beispielsweise Sulfurylchlorid, Thionylchlorid, Thionylbromid, Imide wie z.B. N-Bromsuccinimid, N-Chlorsuccinimid, weiterhin Chlorsulfonsäure aber auch Hypochlorite wie z.B. tert.-Butylhypochlorit in Frage.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen -40°C und 150°C, vorzugsweise zwischen 0°C und 100°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die Halogenierungsmittel im allgemeinen in etwa äquimolaren Mengen ein. Es ist jedoch auch möglich, die eine oder andere Komponente in einem größeren Überschuß (bis zu 3 Mol) zu verwenden.

Das Verfahren (B) ist dadurch gekennzeichnet, dass man Verbindungen der Formel (II), in welcher A, B, Q, W, X, Y und Z die oben angegebenen Bedeutungen haben, in Gegenwart eines Verdünnungsmittels und in Gegenwart eines Nitrierungsmittels umsetzt.

Als Verdünnungsmittel können bei dem erfindungsgemäßen Verfahren (B) alle inerten organischen Solventien eingesetzt werden. Vorzugsweise verwendbar sind halogenierte Kohlenwasserstoffe, wie Methylenchlorid, Chloroform, Dichlorbenzol, Dichlorethan.

Als Nitrierungsmittel kommen "Nitriersäuren" bevorzugt rauchende Salpetersäure in Frage.

Die Reaktionstemperaturen können bei der Durchführung des erfindungsgemäßen Verfahrens (B) innerhalb eines größeren Bereiches variiert werden. Im allgemeinen arbeitet man bei Temperaturen zwischen -50°C und 150°C, vorzugsweise zwischen 0°C und 80°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (II) und das Nitrierungsreagenz im allgemeinen in etwa äquimolaren Mengen ein. Es ist jedoch auch möglich, die eine oder andere Komponente in einem größeren Überschuss (bis zu 5 Mol) zu verwenden.

Die Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren und Nematoden, die in der Landwirtschaft, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella accidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantü, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..

Aus der Ordnung der Siphonaptera z.B. Xenopsylla cheopis, Ceratophyllus spp..

Aus der Klasse der Arachnida z.B. Scorpio maurus, Latrodectus mactans, Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus sinulis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindunsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgerührt werden. Zu den Pflanzenteilen gehört auch Erntegut sowie vegetatives und generatives Vermehrungsmaierial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injezieren und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitem oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol; Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon, (E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid;
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamide,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1 H-inden-1-yl)-1 H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3H)-isobenzofuran]-3'-on,
4-[3,4-Dimethoxyphenyl)-3-(4-fluorphenyl)-acryloyl]-morpholin

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acequinocyl, Acrinathrin, Alanycarb, Aldicarb, Aldoxycarb, Alpha-cypermethrin, Alphamethrin,-Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria, tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos. Dicofol, Diflubenzuron, Dimethoat, Dimethylvinphos, Dinetofuran, Diofenolan, Disulfoton, Docusat-sodium, Dofenapyn,
Eflusilanate, Emamectin; Empenthrin, Endosulfan, Entomopfthora spp., Esfenvalerate, Ethiofencarb, Ethion, Ethiprole, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenthion,, Fenvalerate, Fipronil, Fluazinam, Fluazuron, Flubrocythrinate, Flucycloxuron, Flucythrinate, Flufenoxuron, Flumethrin, Flupyrazofos, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Indoxacarb, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Spirodiclofen, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon Theta-cypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat
N-Cyanomethyl-4-trifluormethyl-nicotinamid
3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren Safenern bzw. Semiochemicals ist möglich. Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew. % Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche
Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari, (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die erfindungsgemäßen Wirkstoffe eignen sich zur Anwendung im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
Sirex juvencus, Urocerus gigas, Urocerus gigas taignus, Urocerus augur.
Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid, Triflumuron, Chlothianidin, Spinosad, Tefluthrin,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
2-*tert.*-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
Benzo[b]thiophencarbonsäurecyclohexylamid-S,S-dioxid, Dichlofluanid, Fluorfolpet, 3-Iod-2-propinyl-butylcarbamat, Tolylfluanid und Azole wie
Azaconazole, Cyproconazole, Epoxyconazole, Hexaconazole, Metconazole, Propiconazole und Tebuconazole;
Molluskizide wie
Fentinacetat, Metaldehyd, Methiocarb, Niclosamid, Thiodicarb und Trimethacarb, Fe-chelate;
   oder herkömmliche Antifouling-Wirkstoffe wie
4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachlorolsophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae; Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coloptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättehen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab. Die erfindungsgemäßen Wirkstoffe können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Hellanthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Frägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpilanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise

Acetochlor, Acifluorfen(-sodium), Aclonifen, Alachlor, Alloxydim(-sodium), Ametryne, Amicarbazone, Amidochlor, Amidosulfuron, Anilofos, Asulam, Atrazine, Azafenidin, Azimsulfuron, Beflubutamid, Benazolin(-ethyl), Benfuresate, Bensulfuron(-methyl), Bentazon, Benzfendizone, Benzobicyclon, Benzofenap, Benzoylprop (-ethyl), Bialaphos, Bifenox, Bispyribac(-sodium), Bromobutide, Bromofenoxim, Bromoxynil, Butachlor, Butafenacil(-allyl), Butroxydim, Butylate, Cafenstrole, Caloxydim, Carbetamide, Carfentrazone(-ethyl), Chlomethoxyfen, Chloramben, Chloridazon, Chlorimuron (-ethyl), Chlomitrofen, Chlorsulfuron, Chlortoluron, Cinidon(-ethyl), Cinmethylm, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen. Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, - Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise: AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arien, wie beispielsweise Venturia inaequalis;
Pyrenophora-Arten, wie beispielsweise Pyrenophora teres oder P. graminea
(Konidienform: Drechslera, Syr: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Im Materialschutz lassen sich die erfindungsgemäßen Stoffe zum Schutz von technischen Materialien gegen Befall und Zerstörung durch unerwünschte Mücroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt: Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicilliuin glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäure-ester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff: vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarbisopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet: Diclomezine; Dicloran; Diethofencarb; Difenoconazole; Diflumetorim; Dimethirimol, Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosinesodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl- 5-thiazolecarboxamide; 2-chloro-N-(2,3-dihydro-1,1,3-trimethyl-1H-inden-4-yl)-3-pyridincarboxam ide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, CisPermethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439,
Eflusilanate, Emamectin, Emamectin-benzoate, Empenthrin (IR-isomer), Endosulfan, Entomopthora spp., EPN, Esfenvalerate, Ethiofencarb, Ethiprole, Ethion, Ethoprophos, Etofenprox, Etoxazole, Etrimfos,
Famphw, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin,
Japonilure,
Kadethrin, Kernpolyederviren, Kinoprene,
Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602, OK-9701, OK-9802, Omethoate, Oxamyl, Oxydemeton-methyl,
Paecilomyces fumosoroseus, Parathion-methyl, Parathion (-ethyl), Permethrin (cis-, trans-), Petroleum, PH-6045, Phenothrin (IR-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos,
Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (IR-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb,
Vamidothion, Vaniliprole, Verbutin, Verticillium lecanii,
WL-108477, WL-40027,
YI-5201, YI-5301, YI-5302,
XMC, Xylylcarb,
ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]-octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze (z.B. gegen Candida-Spezies wie Candida albicans, Candida glabrata ) sowie Epidermophyton floccosum, Aspergillus-Spezies wie Aspergillus niger und Aspergillus fiunigatus, Trichophyton-Spezies wie Trichophyton mentagrophytes,

Microsporon-Spezies wie Microsporon canis und audouinii. Die Aufzählung dieser Pilze stellt keinesfalls eine Beschränkung des erfaßbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwancmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die Herstellung und die Verwendung der erfindungsgemäßen Wirkstoffe geht aus den nachfolgenden Beispielen hervor.

### Herstellungsbeispiele

### Beispiel Nr. 1-1-1

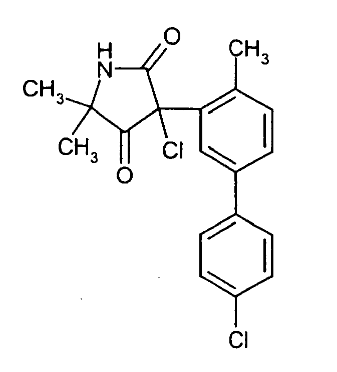

Zu 0,98 g der Verbindung gemäß Beispiel I-1-a-18 (WO 99/48869) in 30 ml absolutem Chloroform werden bei 0 - 5°C 0,24 ml Sulfurylchlorid in 1 ml absolutem Chloroform getropft. Nach 30 min ist die Reaktion beendet (dünnschichtchromatographische Kontrolle).

Das Reaktionsgemisch wird mit NaHCO₃-Lösun gewaschen, die organische Phase getrocknet und das Lösungsmittel abdestilliert.
Ausbeute: 0,85 g (78 % d. Theorie), Fp. 175°C

### Beispiel Nr. I-1-29

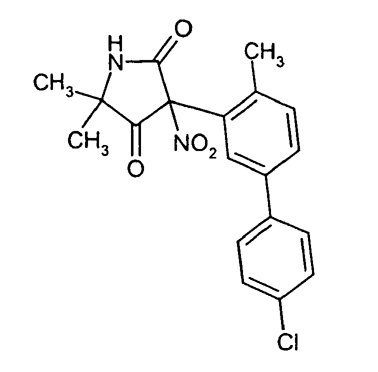

Zu 0,98 g der Verbindung gemäß Beispiel I-1-a-18 (WO 99/48869) in 45 ml absolutem Chloroform werden bei Raumtemperatur 0,33 g (5,2 mmol) rauchende Salpetersäure getropft. Nach 30 min ist die Reaktion beendet (dünnschichtchromatographische Kontrolle).

Das Reaktionsgemisch wird zu 40 ml Eiswasser gegeben, die organische Phase wird abgetrennt, die wäßrige Phase mit Dichlormetan extrahiert und die vereinigten organischen Phasen getrocknet. Anschließend wird das Lösungsmittel abdestilliert. Es erfolgt säulenchromatographische Reinigung an Kieselgel (Dichlormethan, Essigsäureethylester, 10:1).
Ausbeute: 0,75 g (67 % d. Theorie), Fp. 157°C.

In Analogie zu Beispiel (I-1-1) und gemäß den allgemeinen Angaben zur Herstellung wurden folgende Verbindungen der Formel (I-1) erhalten

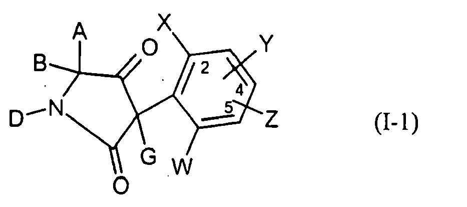

**Tabelle A**

| **Bsp.-Nr.** | **W** | **X** | **Y** | **Z** | **D** | **G** | **A** | **B** | **Fp.°C** |
|---|---|---|---|---|---|---|---|---|---|
| I-1-2 | H | Cl | 5-(4-Cl-C₆H₄) | H | H | Cl | CH₃ | CH₃ | 173 |
| I-1-3 | H | CH₃ | 5-(3-Cl-C₆H₄) | H | H | Cl | CH₃ | CH₃ | 188 |
| I-1-4 | CH₃ | CH₃ | 4-(3-Cl-C₆H₄) | H | H | Cl | (CH₂)₂-CHOCH₃(CH₂)₂- | | 165 |
| I-1-5 | CH₃ | CH₃ | 4-(4-CH₃-C₆H₄) | H | H | Cl | (CH₂)₂-CHOCH₃(CH₂)₂- | | 281 |
| I-1-6 | H | CH₃ | 5-(4-Cl-C₆H₄) | 4-CH₃ | H | Cl | -(CH₂)₂-O-(CH₂)₂- | | 208 |
| I-1-7 | H | CH₃ | 5-(4-Cl-C₆H₄) | 4-CH₃ | H | Cl | -CH₂-O-(CH₂)₃- | | 204 |
| I-1-8 | Cl | CH₃ | 4-(4-Cl-C₆H₄) | H | H | Cl | -CH₂-O-(CH₂)₃- | | 324 |
| I-1-9 | Cl | CH₃ | 4-(4-C1-C₆H₄) | H | H | Cl | -(CH₂)₂-CHOC₂H₅-(CH₂)₂- | | 291 |
| I-1-10 | CH₃ | C₂H₅ | 4-(4-Cl-C₆H₄) | H | H | Cl | -(CH₂)₂-CHOC₂H₅-(CH₂)₂- | | 258 |
| I-1-11 | H | CH₃ | 5-(4-Cl-C₆H₄) | H | H | Cl | i-C₃H₇ | CH₃ | 192 |
| I-1-12 | H | Cl | 5-(3-Cl-C₆H₄) | H | H | Cl | (CH₂)₂-CHOCH₃-(CH₂)₂- | | 228 |
| I-1-13 | H | CH₃ | 5-(3,4-Cl₂-C₆H₃) | H | H | Cl | -(CH₂)₂-CHOCH₃-(CH₂)₂- | | 203 |
| I-1-14 | H | CH₃ | 5-(4-Cl-C₆H₄) | H | H | Cl | -(CH₂)₂-O-(CH₂)₂- | | 218 |
| I-1-15 | H | CH₃ | 5-(3,4-Cl₂-C₆H₃) | H | H | Cl | CH₃ | CH₃ | 222 |
| 1-1-16 | H | Cl | 5-(4-Cl-C₆H₄) | H | H | Cl | -(CH₂)₂-O-(CH₂)₂- | | 250 |
| I-1-17 | H | Cl | 5-(4-Cl-C₆H₄) | H | H | Cl | -(CH₂)₂-CHi-C₃H₇-(CH₂)₂- | | 276 |
| 1-1-18 | H | CH₃ | 5-(4-Cl-C₆H₄) | H | H | Cl | -(CH₂)₂-CHn-C₃H₇-(CH₂)₂- | | 223 |
| 1-1-19 | H | CH₃ | 5-(4-Cl-C₆H₄) | H | H | Cl | -(CH₂)₂-CHi-C₃H₇-(CH₂)₂ | | 245 |
| 1-1-20 | H | Cl | 5-(4-Cl-C₆H₄) | H | H | Cl | -(CH₂)₄- | | 227 |
| 1-1-21 | H | CH₃ | 5-(4-Cl-C₆H₄) | H | H | Cl | -(CH₂)₂- | | 219 |
| 1-1-22 | H | CH₃ | 5-(4-Cl-C₆H₄) | H | H | Cl | -CH₂-CHCH₃-(CH₂)₃- | | 227 |
| I-1-23 | H | Cl | 5-(4-Cl-C₆H₄) | H | H | Cl | -CH₂-CHCH₃-(CH₂)₃- | | 286 |
| 1-1-24 | H | Cl | 5-(4-Cl-C₆H₄) | H | H | Cl | -(CH₂)₅- | | 269 |
| 1-1-25 | H | Cl | 5-(4-Cl-C₆H₄) | H | H | Cl | -(CH₂)₂-CHn-C₃H₇-(CH₂)₂- | | 255 |
| I-1-26 | H | CH₃ | 5-(4-Cl-C₆H₄) | H | H | Cl | -(CH₂)₅- | | 228 |
| 1-1-27 | H | Cl | 5-(4-Cl-C₆H₄) | H | H | Cl | -(CH₂)₂-CHCH₃-(CH₂)₂- | | 290 |
| I-1-28 | H | CH₃ | 5-(4-Cl-C₆H₄) | H | H | Cl | -(CH₂)₄- | | 289 |
| I-1-29 | H | CH₃ | 5-(4-Cl-C₆H₄) | H | H | NO₂ | CH₃ | CH₃ | 157 |

### Beispiel I-2-1

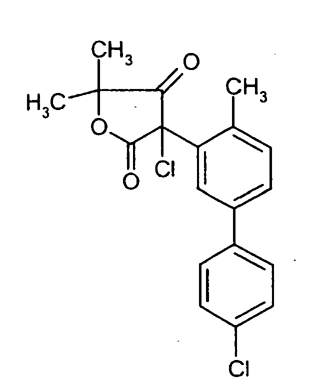

Zu einer Lösung von 0,4 g 3-[2-Methyl-5-(4-chlor-phenyl)-phenyl]-5,5-dimethyl-4-hydroxy-Δ³-furan-2-on in 10 ml absolutem Chloroform tropft man unter Eiskühlung eine Lösung von Sulfurylchlorid (0,164 g) in 10 ml absolutem Chloroform und rührt dann 30 min unter Eiskühlung nach.

Dann wird das Reaktionsgemisch mit Wasser, gesättigter Natriumbicarbonatlösung und gesättigter Kochsalzlösung gewaschen und getrocknet.
Ausbeute: 0,4 g (72 % der Theorie), 10gP (pH 2,5) 4,8.

In Analogie zu Beispiel (I-2-1) und gemäß den allgemeinen Angaben zur Herstellung wurden folgende Verbindungen der Formel (I-2) erhalten

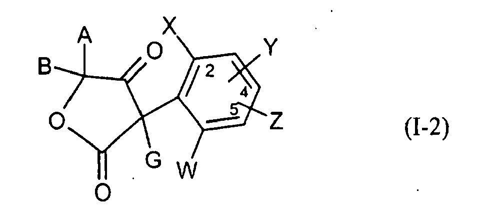

**Tabelle B**

| **Bsp.-Nr.** | **W** | **X** | **Y** | **Z** | **G** | **A** | **B** | **Fp.°C** |
|---|---|---|---|---|---|---|---|---|
| I-2-2 | H | CH₃ | 5-(4-Cl-C₆H₄) | H | Cl | -(CH₂)₂-CHOCH₃-(CH₂)₂ | | 144-146 |
| I-2-3 | H | CH₃ | 5-(4-Cl-C₆H₄) | H | Cl | -(CH₂)₅₋ | | 150 |
| I-2-4 | H | CH₃ | 5-(3-Cl-C₆H₄) | H | Cl | -(CH₂)₂-CHCF₃-(CH₂)₂- | | 108-110 |
| 1-2-5 | H | CH₃ | 5-(4-Cl-C₆H₄) | H | Cl | i-C₃H₇ | CH₃ | logP 5.61 |
| I-2-6 | H | CH₃ | 5-(4-Cl-C₆H₄) | 4-CH₃ | Cl | -(CH₂)₄- | | logP 5.56 |
| 1-2-7 | H | C₂H₅ | 5-(4-Cl-C₆H₄) | H | Cl | CH₃ | CH₃ | logo 5.05 |
| 1-2-8 | H | CH₃ | 5-(4-CN-C₆H₄) | H | Cl | CH₃ | CH₃ | 198 |
| 1-2-9 | H | CH₃ | 5-(4-CN-C₆H₄) | H | Cl | -(CH₂)₄- | | 196 |
| 1-2-10 | H | CH₃ | 5-(4-F-C₆H₄) | H | Cl | CH₃ | CH₃ | 133-135 |
| I-2-11 | H | CH₃ | 5-(3-F-C₆H₄) | H | Cl | CH₃ | CH₃ | log P 4.30 . |
| I-2-12 | CH₃ | CH₃ | 4-(4-Cl-C₆H₄) | H | Cl | -(CH₂)₅- | | 262 |
| I-2-13 | CH₃ | C₂H₅ | 4-(4-Cl-C₆H₄) | H | Cl | -(CH₂)₂-CHOC₂H₅-(CH₂)₂- | | log P 5.59 |
| I-2-14 | CH₃ | CH₃ | 4-(4-Cl-C₆H₄) | H | Cl | CH₃ | CH₃ | log P 5.11 |
| I-2-15 | CH₃ | CH₃ | 4-(4-Cl-C₆H₄) | H | Cl | -(CH₂)₄- | | 152-155 |
| 1-2-16 | CH₃ | CH₃ | 4-(4-Cl-C₆H₄) | H | Cl | -(CH₂)₂-CHCH₃-(CH₂)₂- | | 85-88 . |
| I-2-17 | CH₃ | CH₃ | 4-(4-Cl-C₆H₄) | H | Cl | -CH₂-O-(CH₂)₃- | | log P 4.91 1 |
| I-2-18 | CH₃ | CH₃ | 4-(4-Cl-C₆H₄) | H | Cl | -(CH₂)₂-O-(CH₂)₂- | | 178-180 |

| | | | | | | | | |
|---|---|---|---|---|---|---|---|---|
| logP-Bestimmung bei pH = 2,3 | | | | | | | | |

Die Bestimmung der angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1 % wässrige Phosphorsäure, Acetonitril; linearer Gradient von 10 % Acetonitril bis 90 % Acetonitril.
(b) Eluenten für die Bestimmung im neutralen Bereich: 0,01-molare wässrige Phosphatpuffer-Lösung, Acetonitril; linearer Gradient von 10 % Acetonitril bis 90 % Acetonitril.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenen Alkanonen).

Die lambda-max-Werte wurde an Hand der UV-Spektren von 200 nm bis 400 nm in den Maxima der chromatographischen Signale ermittelt.

### Beispiel 1-3-1

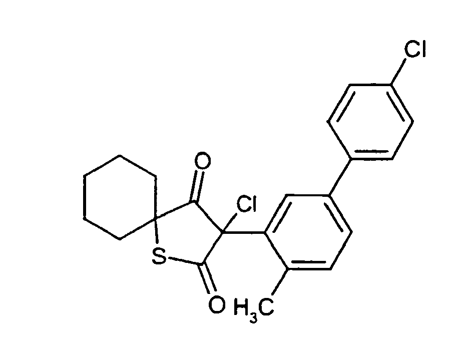

Zu 0,385 g des Beispiels I-3-a-1 (WO 99/48869) in 10 ml absolutem Chloroform werden unter Eiskühlung 0,10 ml Sulfurylchlorid in 0,5 ml absolutem Chloroform getropft. Es wird 20 min gerührt.

Das Reaklionsgemisch wird mit NaHCO₃-Lösun gewaschen, die organische Phase getrocknet und das Lösungsmittel abdestilliert. Zur weiteren Reinigung wird das Produkt mehrfach mit Cyclohexan/Essigsäureethylester verrührt.
Ausbeute: 0,315 g (75 % d. Theorie), Fp. 157 - 160°C

### Tabelle C

In Analogie zu Beispiel (I-3-1) und gemäß den allgemeinen Angaben zur Herstellung wurden folgende Verbindungen der Formel (I-3) erhalten:

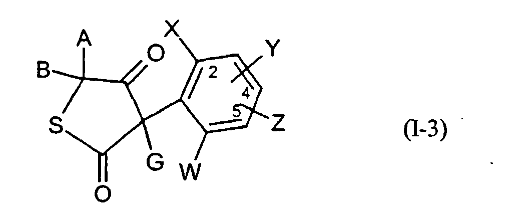

| **Bsp.-Nr.** | **W** | **X** | **Y** | **Z** | **G** | **A** | **B** | **Fp°C** |
|---|---|---|---|---|---|---|---|---|
| I-3-2 | H | CH₃ | 5-(4-Cl-C₆H₄) | 4-CH₃ | Cl | -(CH₂)₅- | | Wachs |

### Beispiel A

### Myzus-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea), die stark von der Grünen Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele gute Wirksamkeit:

**Tabelle A**

| pflanzenschädigende Insekten | | |
|---|---|---|
| **Myzus persicae -Test** | | |
| Wirkstoffe | Wirkstoffkonzentration in ppm | Abtötungsgrad in % nach 6^{d} |
| Bsp. I-1-1 | 500 | 95 |
| Bsp. I-1-14 | 500 | 100 |
| Bsp. I-1-6 | 500 | 98 |
| Bsp. I-1-7 | 500 | 100 |
| Bsp. I-1-9 | 500 | 95 |
| Bsp. I-1-13 | 500 | 100 |
| Bsp I-1-15 | 500 | 95 |

### Beispiel B

### Phaedon-Larven-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit:

**Tabelle B**

| pflanzenschädigende Insekten | | |
|---|---|---|
| **Phaedon-Larven -Test** | | |
| Wirkstoff | Wirkstoffkonzentration in ppm | Abtötungsgrad in % nach 7^{d} |
| Bsp. I-2-1 | 1000 | 100 |
| Bsp. I-1-2 | 500 | 100 |
| Bsp. I-1-1 | 500 | 100 |
| Bsp. I-1-3 | 500 | 100 |
| Bsp. I-1-14 | 500 | 100 |
| Bsp. I-1-6 | 500 | 100 |
| Bsp. I-1.7 | 500 | 100 |
| Bsp. I-1-9 | 500 | 100 |
| Bsp. I-1-11 | 500 | 100 |
| Bsp. I-1-12 | 500 | 100 |
| Bsp. I-1-13 | 500 | 100 |
| Bsp. I-1-15 | 500 | 100 |
| Bsp. I-3-1 | 500 | 100 |

### Beispiel C

### Plutella-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z.B. die folgenden Verbindungen der Herstellungsbeispiele gute Wirksamkeit:

**Tabelle C**

| pflanzenschädigende Insekten | | |
|---|---|---|
| **Plutella-Test** | | |
| Wirkstoff | Wirkstoffkonzentration in ppm | Abtötungsgrad in % nach 7^{d} |
| Bsp. I-1-11 | 500 | 100 |
| Bsp. I-1-12 | 500 | 100 |
| Bsp. 1-1-13 | 500 | 100 |
| Bsp. 1-1-15 | 500 | 100 |

### Beispiel D

### Spodoptera exigua-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera exigua) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z.B. die folgenden Verbindungen der Herstellungsbeispiele gute Wirksamkeit:

**Tabelle D**

| pflanzenschädigende Insekten | | |
|---|---|---|
| **Spodoptera exigua-Test** | | |
| Wirkstoffe | Wirkstoffkonzentration in ppm | Abtötungsgrad in % nach 7^{d} |
| Bsp. I-1-11 | 500 | 90 |
| Bsp. I-1-13 | 500 | 100 |
| Bsp. I-1-15 | 500 | 100 |

### Beispiel E

### Spodoptera frugiperda-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele gute Wirksamkeit:

**Tabelle E**

| pflanzenschädigende Insekten | | |
|---|---|---|
| **Spodoptera frugiperda-Test** | | |
| Wirkstoffe | Wirkstoffkonzentration in ppm | Abtötungsgrad in % nach 7^{d} |
| Bsp. I-2-1 | 1000 | 100 |
| Bsp. I-1-2 | 500 | 100 |
| Bsp. I-1-1 | 500 | 100 |
| Bsp. I-1-14 | 500 | 100 |
| Bsp. I-1-6 | 500 | 100 |
| Bsp. I-1-7 | 500 | 100 |
| Bsp.I-1-11 | 500 | 100 |
| Bsp. I-1-12 | 500 | 100 |
| Bsp. I-1-13 | 500 | 100 |
| Bsp. I-1-15 | 500 | 100 |

### Beispiel F

### Tetranychus-Test (OP-resistent/Tauchbehandlung)

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele gute Wirksamkeit:

**Tabelle F**

| pflanzenschädigende Milben | | |
|---|---|---|
| **Tetranychus-Test** (OP-resistent/Tauchbehandlung) | | |
| Wirkstoff | Wirkstoffkonzentration in ppm | Abtötungsgrad in % nach 7^{d} |
| Bsp. I-2-1 | 100 | 98 |
| Bsp. I-1-2 | 100 | 100 |
| Bsp. I-1-1 | 100 | 100 |
| Bsp. I-1-14 | 100 | 99 |
| Bsp. I-1-6 | 100 | 99 |
| Bsp. I-1-7 | 100 | 98 |
| Bsp. I-1-8 | 100 | 98 |
| Bsp. I-1-11 | 100 | 99 |
| Bsp. I-1-12 | 100 | 100 |
| Bsp. I-I-13 | 100 | 95 |
| Bsp. I-1-15 | 100 | 99 |

### Beispiel G

### In vitro-Test zur ED₅₀-Bestimmung bei Mikroorganismen

In die Kavitäten von Mikrotiterplatten wird eine methanolische Lösung des zu prüfenden Wirkstoffs, versetzt mit dem Emulgator PS16, pipettiert. Nachdem das Lösungsmittel abgedampft ist, werden je Kavität 200 µl Potatoe-Dextrose-Medium hinzugefügt.

Das Medium wurde vorher mit einer geeigneten Konzentration von Sporen bzw. Mycel des zu prüfenden Pilzes versetzt.

Die resultierenden Konzentrationen des Wirkstoffs betragen 0,1, 1, 10 und 100 ppm. Die resultierende Konzentration des Emulgators beträgt 300 ppm.

Die Platten werden anschließend 3-5 Tage auf einem Schüttler bei einer Temperatur von 22°C inkubiert, bis in der unbehandelten Kontrolle ein ausreichendes Wachstum feststellbar ist.

Die Auswertung erfolgt photometrisch bei einer Wellenlänge von 620 nm. Aus den Messdaten der verschiedenen Konzentrationen wird die Wirkstoffdosis, die zu einer 50 %igen Hemmung des Pilzwachstums gegenüber der unbehandelten Kontrolle führt (ED₅₀), berechnet.

**Tabelle G**

| **In vitro-Test** zur ED₅₀-Bestimmung bei Mikroorganismen | | |
|---|---|---|
| Wirkstoff | Mikroorganismus | ED₅₀-Wert |
| Bsp. I-3-1 | Botrytis cinerea | <0,10 |
| | Pyricularia oryzae | <0,10 |
| | Septoria tritici | 0,61 |
| | Ustilago avenae | 0,27 |

### Beispiel H

### Post-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffinengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

### Beispiel I

### Pre-emergence-Test

| | |
|---|---|
| Lösungsmittel: | 5 Gewichtsteile Aceton |
| Emulgator: | 1 Gewichtsanteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung bespritzt so, dass die jeweils gewünschten Wirkstoffmengen pro Flächeneinheit ausgebracht werden. Die Konzentration der Spritzbrühe wird so ausgewählt, dass in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % - | keine Wirkung (wie unbehandelte Kontrolle) |
| 100 % = | totale Vernichtung |

| pre-emergence/ Gewächshaus | g ai/ha | Alopecurus | Avena fatua | Echinochloa | | Setaria | Amaranthus |
|---|---|---|---|---|---|---|---|
| Bsp.I-1-4 | 250 | 95 | 90 | | 100 | 100 | 90 |
| post-emergence/ Gewächshaus | g ai/ha | Zuckerrüben | Alopecurus | | Avena fatua | Echinochloa | Setaria |
| Bsp. I-1-14 | 250 | 0 | 80 | | 100 | 100 | 100 |
| Bsp. I-1-11 | | 250 0 | 95 | | 90 | 100 | 100 |
| post-emergence / Gewächshaus | g ai/ha | Alopecurus | Avena fatua | | Echinochloa | Setaria | |
| Bsp. I-1-7 | 250 | 90 | 95 | | 100 | 100 | |
| Bsp. I-1-9 | 250 | 95 | 100 | | 100 | 100 | |
| Bsp. I-1-13 | 250 | 95 | 90 | | 100 | 90 | |
| post-emergence / Gewächshaus | g ai/ha | Zuckerrüben | Alopecurus | Avena fatua | Digitaria | Echinochloa | Setaria |
| Bsp. I-1-2 | 125 | 0 | 90 | 95 | 100 | 100 | 90 |
| Bsp. I-1-1 | 125 | 0 | 95 | 100 | 90 | 100 | 100 |
| post-emergence/ Gewächshaus | g ai/ha | Alopecurus | Avena fatua | Echinochloa | Setaria | Amaranthus | Sinapis |
| Bsp. I-1-6 | 250 | 70 | 100 | 100 | 95 | 70 | |
| Bsp. I-1-8 | 250 | 95 | 95 | 100 | 100 | 70 | 80 |
| Bsp. I-1-10 | 250 | 100 | 100 | 100 | 100 | 80 | 80 |
| Bsp. I-1-4 | 250 | 100 | 100 | 100 | 100 | - | 70 |

### Beispiel J

### Plasmopara-Test (Rebe) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei 20°C und 100 % relativer Luftfeuchtigkeit. Anschließend werden die Pflanzen 4 Tage im Gewächshaus bei ca. 21°C und ca. 90 % Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle J Plasmopara-Test (Rebe) / protektiv**

| Wirkstoff | Aufwandmenge an Wirkstoff in g/ha | % Wirkungsgrad |
|---|---|---|
| Bsp. I-1-2 | 100 | 100 |
| Bsp.I-1-1 | 100 | 100 |
| Bsp.I-1-14 | 100 | 100 |
| Bsp. I-1-6 | 100 | 94 |
| Bsp. I-1-11 | 100 | 100 |
| Bsp. I-1-12 | 100 | 94 |
| Bsp. I-1-13 | 100 | 94 |

### Beispiel K

### Venturia-Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle K V**

| Venturia-Test (Apfel) / protektiv | | |
|---|---|---|
| Wirkstoff | Aufwandmenge an Wirkstoff in g/ha | % Wirkungsgrad |
| Bsp. I-1-2 | 100 | 96 |
| Bsp. I-1-1 | 100 | 97 |
| Bsp.I-1-6 | 100 | 99 |
| Bsp. I-1-11 | 100 | 100 |

### Beispiel L

### Grenzkonzentrations-Test / Bodeninsekten - Behandlung transgener Pflanzen

| | |
|---|---|
| Testinsekt: | Diabrotica balteata - Larven im Boden |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und läßt diese bei 20°C stehen.

Sofort nach dem Ansatz werden je Topf 5 vorgekeimte Maiskömer der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel M

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten, bestimmt.

## Patentansprüche

1. Verbindungen der Formel (I), 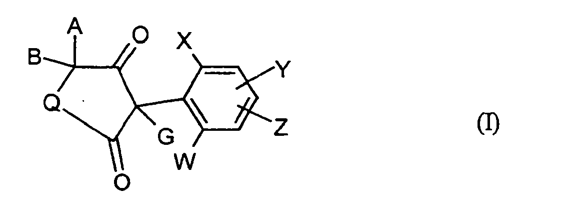 in welcher
Q für Sauerstoff, Schwefel oder die Gruppe N-D steht,
W für Wasserstoff, Halogen, C₁-C₆-Alkyl oder C₁-C₆-Alkoxy steht,
X für Halogen, C₁-C₆₋Alkyl, C₁-C₆-Halogenalkyl, C₁-C₆-Alkoxy, C₁- C₆-Halogenalkoxy, Cyano oder gegebenenfalls durch Halogen, C₁- C₆-Alkyl; C₁-C₆-Alkoxy, C₁-C₄-Halogenalkyl, C₁-C₄-Halogen- alkoxy, Nitro oder Cyano substituiertes Phenyl steht,
Y für einen der Reste 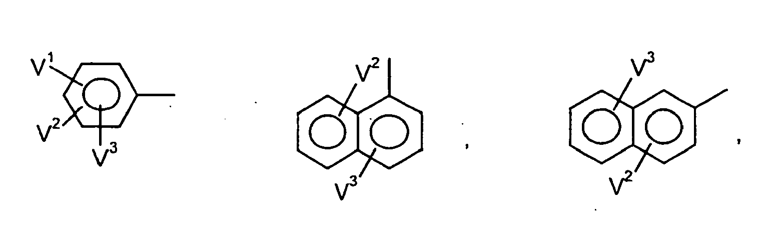 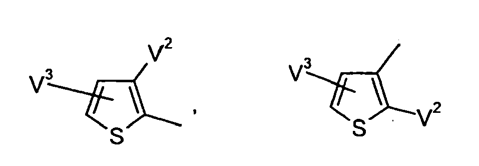
steht,
V¹ für Wasserstoff, Halogen, C₁-C₁₂-Alkyl, C₁-C₆-Alkoxy, C₁-C₆- Alkylthio, C₁-C₆-Alkylsulfinyl, C₁-C₆-Alkylsulfonyl, C₁-C₄-Halogen- alkyl, C₁-C₄-Halogenalkoxy, Nitro, Cyano oder gegebenenfalls einfach bis zweifach durch Halogen, C₁-C₆-Alkyl, C₁-C₆-Alkoxy, C₁- C₄-Halogenalkyl, C₁-C₄-Halogenalkoxy, Nitro oder Cyano substituiertes Phenyl steht,
V² und V³ unabhängig voneinander für Wasserstoff, Halogen, C₁-C₆-Alkyl, C₁-C₆-Alkoxy, C₁-C₄-Halogenalkyl oder C₁-C₄-Halogenalkoxy stehen,
Z für Wasserstoff, Halogen, C₁-C₆-Alkyl, C₁-C₆-Halogenalkyl, C₁-C₆- Alkoxy, C₁-C₆-Halogenalkoxy, Nitro oder Cyano steht,
A für jeweils gegebenenfalls durch Halogen substituiertes C₁-C₁₂-Alkyl, C₃-C₈-Alkenyl, C₁-C₁₀-Alkoxy-C₁-C₈-alkyl, Poly-C₁-C₈-alkoxy-C₁- C₈-alkyl, C₁-C₁₀-Alkylthio-C₁-C₆-alkyl, gegebenenfalls durch Halo- gen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆- Halogenalkyl, C₁-C₆-Alkoxy, C₁-C₆-Halogenalkoxy, Cyano oder Nitro substituiertes Phenyl, Phenyl-C₁-C₆-alkyl oder für Wasserstoff steht, wenn im Falle der Gruppe (I-1) D ungleich Wasserstoff ist,
B für Wasserstoff, C₁-C₁₂-Alkyl oder C₁-C₈-Alkoxy-C₁-C₆-alkyl steht oder
A, B und das Kohlenstoffatom an das sie gebunden sind, für gesättigtes C₃- C₁₀-Cycloalkyl oder ungesättigtes C₅-C₁₀-Cyclolkyl stehen, worin gegebenenfalls ein Ringglied durch Sauerstoff oder Schwefel ersetzt ist und welche gegebenenfalls einfach oder zweifach durch C₁-C₈- Alkyl, C₃-C₁₀₋Cycloalkyl, C₁-C₆-Halogenalkyl, C₁-C₈-Alkoxy, C₁- Cg-Alkylthio, Halogen oder Phenyl substituiert sind oder
A, B und das Kohlenstoffatom, an das sie gebunden sind, für C₃-C₆- Cycloalkyl stehen, welches durch eine gegebenenfalls ein oder zwei nicht direkt benachbarte Sauerstoff- und/oder Schwefelatome enthaltende Alkylendiyl-, oder durch eine Alkylendioxyl- oder durch eine Alkylendithioyl-Gruppe substituiert ist, die mit dem Kohlen- stoffatom, an das sie gebunden ist, einen weiteren fünf- bis acht- gliedrigen Ring bildet oder
A, B une das Kohlenstoffatom, an das sie gebunden sind, für C₃-C₈- Cycloalkyl oder C₅-C₈-Cycloalkenyl stehen, in welchen zwei Sub- stituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Allcandiendiyl stehen, worin gegebenenfalls eine Methylengruppe durch Sauerstoff oder Schwefel ersetzt ist,
D für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂- C₈-akyl, Poly-C₁-C₈-alkoxy-C₂-C₈-alkyl, C₁-C₁₀-Alkylthio-C₂-C₈- alkyl, gegebenenfalls durch Halogen, C₁-C₄-Alkyl, C₁-C₄-Alkoxy oder C₁-C₄-Halogenalky substituiertes C₃-C₈-Cycloalkyl, in welchem gegebenenfalls ein Ringglied durch Sauerstoff oder Schwefel ersetzt ist oder gegebenenfalls durch Halogen, C₁-C₆-Alkyl, C₁-C₆- Halogenalkyl, C₁-C₆-Allcoxy, C₁-C₆-Halogenalkoxy, Cyano oder Nitro substituiertes Phenyl steht,
A und D gemeinsam für jeweils gegebenenfalls substituiertes C₃-C₆- Alkandiyl oder C₃-C₆-Alkendiyl stehen, worin gegebenenfalls eine Methylengruppe durch Sauerstoff oder Schwefel ersetzt ist und wobei als Substituenten jeweils in Frage kommen: C₁-C₆-Alkyl, C₁-C₆-Alkoxy oder eine weitere C₃-C₆-Alkandiyl- gruppierung,
G für Chlor, Brom oder Nitro steht.

2. Verbindungen der Formel (I) gemäß Anspruch 1, in welcher
Q für Sauerstoff, Schwefel oder die Gruppe N-D steht,
W für Wasserstoff, Chlor, Brom oder C₁-C₄-Alkyl steht,
X für Fluor, Chlor, Brom, C₁-C₄-Alkyl, C₁-C₄-Alkoxy, C₁-C₄- Halogenalkyl, C₁-C₄-Halogenalkoxy oder Cyano steht,
Y für den Rest 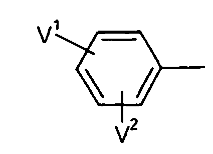
steht,
V¹ für Wasserstoff, Fluor, Chlor, Brom, C₁-C₆-Alkyl, C₁-C₄-Alkoxy, C₁- C₂-Halogenalkyl, C₁-C₂-Halogenakoxy, Nitro oder Cyano, gegebe- nenfalls einfach oder zweifach durch Fluor, Chlor, Brom, C₁-C₄- Alkyl, C₁-C₄-Alkoxy, C₁-C₂-Halogenalkyl, C₁-C₂-Halogenalkoxy, Nitro oder Cyano substituiertes Phenyl steht,
V² für Wasserstoff, Fluor, Chlor, Brom, C₁-C₄-Alkyl, C₁-C₄-Alkoxy, C₁- C₂-Halogenalkyl oder C₁-C₂-Halogenalkoxy steht,
Z für Wasserstoff, Fluor, Chlor, Brom, C₁-C₄-Alkyl, C₁-C₂-Halogen- alkyl, C₁-C₄-Alkoxy oder C₁-C₂-Halogenalkoxy steht,
A für jeweils gegebenenfalls einfach bis fünffach durch Fluor oder Chlor substituiertes C₁-C₁₀-Alkyl, C₁-C₈-Alkoxy-C₁-C₆-alkyl, gegebenen- falls einfach bis zweifach durch Fluor, Chlor, C₁-C₄-Alkyl oder C₁- C₄-Alkoxy substituiertes C₃-C₇-Cycloalkyl, in welchem gegebenen- falls ein Ringglied durch Sauerstoff oder Schwefel ersetzt ist oder jeweils gegebenenfalls einfach bis zweifach durch Fluor, Chlor, Brom, C₁-C₄-Alkyl, C₁-C₄-Halogenalkyl, C₁-C₄-Alkoxy oder C₁-C₄-Halo- genalkoxy substituiertes Phenyl oder Phenyl-C₁-C₄-alkyl oder für Wasserstoff steht, wenn im Falle der Gruppe (I-1) D ungleich Wasserstoff ist,
B für Wasserstoff oder C₁-C₆-Alkyl steht, oder
A, B und das Kohlenstoffatom an das sie gebunden sind, für gesättigtes C₃- C₈-Cycloakyl stehen, worin gegebenenfalls ein Ringglied durch Sauerstoff oder Schwefel ersetzt ist und welches gegebenenfalls einfach bis zweifach durch C₁-C₆-Alkyl, C₅-C₆-Cycloakyl, C₁-C₃- Halogenalkyl, C₁-C₆-Alkoxy, Fluor, Chlor oder Phenyl substituiert ist, mit der Maßgabe, dass D in der Gruppe (I-1) für Wasserstoff oder C₁-C₄-Alkyl steht,
D für Wasserstoff, für jeweils gegebenenfalls einfach bis fünffach durch Fluor oder Chlor substituiertes C₁-C₁₀-Alkyl, C₃-C₆-Alkenyl, C₁- C₄-Alkoxy-C₂-C₄-alkyl oder C₁-C₄-Alkylthio-C₂-C₄-alkyl, für gege- benenfalls einfach bis zweifach durch Fluor, Chlor, C₁-C₄-Alkyl, C₁- C₄-Alkoxy oder C₁-C₂-Halocenalkyl substituiertes C₃-C₇-Cycloal- kyl, in welchem gegebenenfalls eine Methylengruppe durch Sauerstoff oder Schwefel ersetzt ist oder für gegebenenfalls einfach bis zweifach durch Fluor, Chlor, Brom, C₁-C₄-Alkyl, C₁-C₄-Halogenalkyl, C₁-C₄- Alkoxy oder C₁-C₄-Halogenalkoxy substituiertes Phenyl steht, oder
A und D gemeinsam für gegebenenfalls substituiertes C₃-C₅-Alkandiyl stehen, in welchem eine Methylengruppe durch Sauerstoff oder Schwefel ersetzt sein kann, wobei als Substituenten C₁-C₄-Alkyl in Frage kommen,
G für Chlor, Brom oder Nitro steht.

3. Verbindungen der Formel (I) gemäß Anspruch 1, in welcher.
Q für Sauerstoff, Schwefel oder die Gruppe N-D steht,
W für Wasserstoff, Chlor, Methyl oder Ethyl steht,
X für Chlor, Methyl, Ethyl, n-Propyl, iso-Propyl, Methoxy, Ethoxy, n- Propoxy, iso-Propoxy, Trifluormethyl, Difluormethoxy, Trifluor- methoxy oder Cyano steht,
Y für den Rest 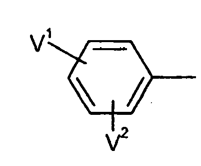
steht,
V¹ für Wasserstoff, Fluor, Chlor, Brom, Methyl, Ethyl, n-Propyl, iso- Propyl, n-Butyl, iso-Butyl, tert.-Butyl, Methoxy, Ethoxy, n-Propoxy, iso-Propoxy, Trifluormethyl, Trifluormethoxy, Nitro oder Cyano steht,
V² für Wasserstoff, Fluor, Chlor, Methyl, Ethyl, n-Propyl, iso-Propyl, Methoxy, Ethoxy, Trifluormethyl oder Trifluormethoxy steht,
Z für Wasserstoff, Fluor, Chlor, Methyl, Ethyl, n-Propyl oder Methoxy steht,
A für C₁-C₆-Alkyl oder C₁-C₂-Alkoxy-C₁-C₂-alkyl, C₃-C₆-Cycloalkyl, in welchem Gegebenenfalls ein Ringglied durch Sauerstoff oder Schwefel ersetzt ist oder für Wasserstoff steht, wenn im Falle der Gruppe (I-1) D ungleich Wasserstoff ist,
B für Wasserstoff, Methyl, Ethyl steht oder
A, B und das Kohlenstoffatom an das sie gebunden sind, für gesättigtes C₃- C₈-Cycloakyl stehen, in welchem gegebenenfalls ein Ringglied durch Sauerstoff oder Schwefel ersetzt ist und welches gegebenenfalls einfach durch Methyl, Ethyl, n-Propyl, Isopropyl, n-Butyl, Isobutyl, tert.-Butyl, Trifluormethyl, Methoxy, Ethoxy, n-Propoxy, iso-Propoxy, n-Butoxy oder iso-Butoxy substituiert ist, mit der Maßgabe, dass D in der Gruppe (I-1) für Wasserstoff oder C₁-C₃-Alkyl steht,
D für Wasserstoff, Methyl, Ethyl, n-Propyl, Isopropyl, Cyclopropyl, Cyclopentyl oder Cyclohexyl steht,
A, D gemeinsam für gegebenenfalls substituiertes C₃-C₄-Alkandiyl stehen, worin gegebenenfalls ein Kohlenstoffatom durch Sauerstoff oder Schwefel ersetzt ist,
G für Chlor oder Nitro steht.

4. Verbindungen der Formel (I) gemäß Anspruch 1, in welcher
Q für die Gruppe N-D steht,
W für Wasserstoff, Chlor oder Methyl steht,
X für Chlor, Methyl oder Ethyl steht,
Y für den Rest 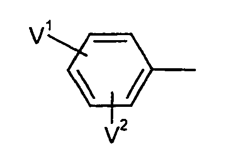
steht,
V¹ für Chlor oder Methyl steht,
V² für Wasserstoff oder Chlor steht,
Z für Wasserstoff oder Methyl steht,
A für Methyl, Ethyl, Propyl, Isopropyl, n-Butyl, Isobutyl, s-Butyl, tert.- Butyl, Cyclopropyl, Cyclopentyl oder Cyclohexyl steht,
B für Methyl steht,
A,B und das Kohlenstoffatom, an das sie gebunden sind, für gesättigtes C₃- C₈-Cycloakyl steht, in welchem gegebenenfalls ein Ringglied durch Sauerstoff oder Schwefel ersetzt ist und welches gegebenenfalls ein- fach durch Methyl oder im Falle von C₆-Cycloalkyl auch durch Ethyl, n-Propyl, Isopropyl, n-Butyl, Isobutyl, tert.-Butyl, Trifluormethyl, Methoxy, Ethoxy, n-Propoxy, iso-Propoxy, n-Butoxy oder iso-Butoxy substituiert ist,
D für Wasserstoff steht,
G für Chlor oder Nitro steht.

5. Verbindungen der Formel (I) gemäß Anspruch 1, in welcher
Q für Sauerstoff steht,
W für Wasserstoff, Chlor oder Methyl steht,
X für Chlor, Methyl oder Ethyl steht,
Y für den Rest 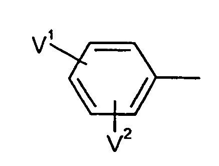
steht,
V¹ für Chlor, Fluor, Cyano oder Methyl steht,
V² für Wasserstoff oder Chlor steht,
Z für Wasserstoff oder Methyl steht,
A für Methyl, Ethyl, Propyl, Isopropyl, n-Butyl, Isobutyl, s-Butyl, tert.- Butyl, Cyclopropyl, Cyclopentyl oder Cyclohexyl steht,
B für Methyl steht oder
A, B und das Kohlenstoffatom an das sie gebunden sind, für gesättigtes C₃- C₈-Cycloalkyl steht, in welchem gegebenenfalls ein Ringglied durch Sauerstoff oder Schwefel ersetzt ist und welches gegebenenfalls einfach durch Methyl oder im Falle von C₆-Cycloalkyl auch durch Ethyl, n-Propyl, Isopropyl, n-Butyl, Isobutyl, tert.-Butyl, Tri- fluormethyl, Methoxy, Ethoxy, n-Propoxy, iso-Propoxy, n-Butoxy oder iso-Butoxy substituiert ist,
G für Chlor steht.

6. Verbindungen der Formel (I) gemäß Anspruch 1, in welcher
Q für Schwefel steht,
W für Wasserstoff steht,
X für Chlor oder Methyl steht,
Y für den Rest 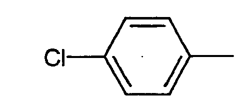
steht ,
Z für Wasserstoff oder Methyl steht,
A, B und das Kohlenstoffatom, an das sie gebunden sind, für gesättigtes C₅- C₆-Cycloalkyl steht, in welchem gegebenenfalls ein Ringglied durch Sauerstoff ersetzt ist und welches gegebenenfalls durch Methyl oder Methoxy substituiert ist,
G für Chlor steht.

7. Verfahren zur Herstellung von Verbindungen der Formel (I) gemäß Anspruch 1, **dadurch gekennzeichnet, dass** man zum Erhalt von
A) Verbindungen der Formeln (I-1) bis (I-3) 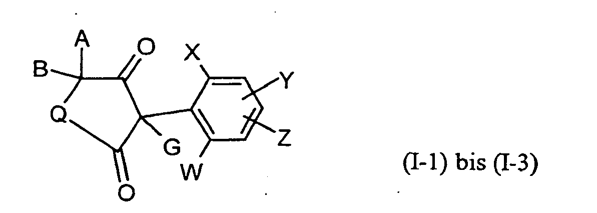 in welcher
A, B, Q, W; X, Y und Z, die oben angegebene Bedeutung haben
und
G für Halogen steht,
Verbindungen der Formeln (D-1) bis (II-3) 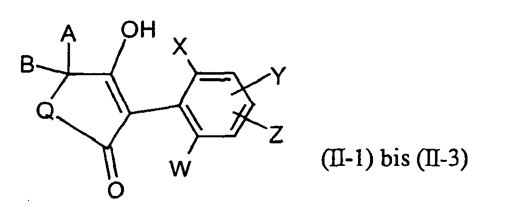 in welcher
A, B, Q, W, X, Y und Z die oben angegebene Bedeutung haben
mit Halogenierungsmittel in Gegenwart eines Lösungsmittels und gegebenenfalls in Gegenwart eines Radikalstarters umsetzt oder
B) Verbindungen der Formeln (I-1) bis (I-3) 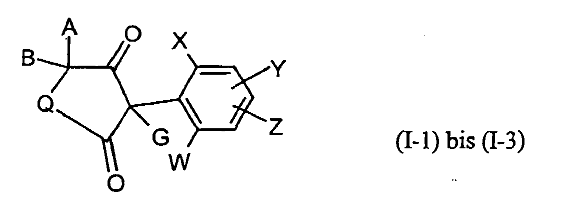 in welcher
A, B, Q, W, X, Y und Z die oben angegebene Bedeutung haben und
G für Nitro steht,
Verbindungen der Formeln (II-1) bis (II-3) 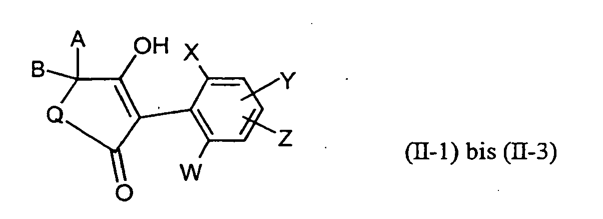 in welcher
A, B, Q, W, X, Y und Z die oben angegebene Bedeutung haben mit Nitrierungsreagenzien in Gegenwart eines Lösungsmittels umsetzt.

8. Schädlingsbekämpfungsmittel und/oder Herbizide und/oder Fungizide, **gekennzeichnet durch** einen Gehalt an mindestens einer Verbindung der Formel (I) gemäß Anspruch 1.

9. Verbindungen der Formel (I) gemäß Anspruch 1 zur Verwendung in Verfahren zur Bekämpfung von tierischen Schädlingen und/oder unerwünschten Pflanzenbewuchs und/oder Pilzen, **dadurch gekennzeichnet, dass** man Verbindungen der Formel (I) gemäß Anspruch 1 auf Schädlinge und/oder ihren Lebensraum einwirken läßt.

10. Verbindungen der Formel (I) gemäß Anspruch 1 zur Verwendung zur Bekämpfung von tierischen Schädlingen und/oder unerwünschtem Pflanzenbewuchs und/oder Pilzen.

11. Verfahren zur Herstellung von Schädlingsbekämpfungsmitteln und/oder Herbiziden und/oder Fungiziden, **dadurch gekennzeichnet, dass** man Verbindungen der Formel (I) gemäß Anspruch 1 mit Streckmitteln und/oder oberflächenaktiven Stoffen vermischt.

12. Verwendung von Verbindungen der Formel (I) gemäß Anspruch 1 zur Herstellung von Schädlingsbekämpfungsmitteln und/oder Herbiziden und/oder Fungiziden.

## Claims

1. Compounds of the formula (I) 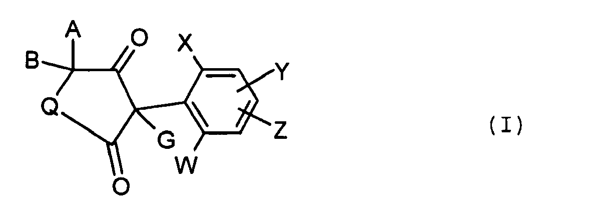 in which
Q represents oxygen, sulphur or the group N-D,
W represents hydrogen, halogen, C₁-C₆-alkyl or C₁-C₆-alkoxy,
X represents halogen, C₁-C₆-alkyl, C₁-C₆- haloalkyl, C₁-C₆-alkoxy, C₁-C₆-haloalkoxy, cyano or optionally halogen-, C₁-C₆-alkyl-, C₁-C₆-alkoxy-, C₁-C₄-haloalkyl-, C₁-C₄- haloalkoxy-, nitro- or cyano-substituted phenyl,
Y represents one of the radicals 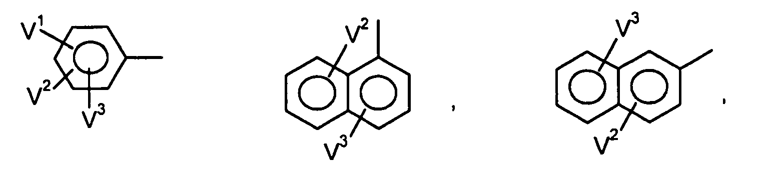 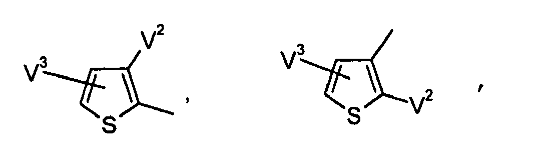
V¹ represents hydrogen, halogen, C₁-C₁₂-alkyl, C₁-C₆-alkoxy, C₁-C₆-alkylthio, C₁-C₆- alkylsulphinyl, C₁-C₆-alkylsulphonyl, C₁-C₄- haloalkyl, C₁-C₄-haloalkoxy, nitro, cyano or phenyl which is optionally mono- or disubstituted by halogen, C₁-C₆-alkyl, C₁-C₆- alkoxy, C₁-C₄-haloalkyl, C₁-C₄-haloalkoxy, nitro or cyano,
V² and V³ independently of one another represent hydrogen, halogen, C₁-C₆-alkyl, C₁-C₆-alkoxy, C₁-C₄-haloalkyl or C₁-C₄-haloalkoxy,
Z represents hydrogen, halogen, C₁-C₆-alkyl, C₁-C₆-haloalkyl, C₁-C₆-alkoxy, C₁-C₆- haloalkoxy, nitro or cyano,
A represents in each case optionally halogen- substituted C₁-C₁₂-alkyl, C₃-C₈-alkenyl, Cₗ-C₁₀-alkoxy-C₁-C₈-alkyl, poly-C₁-C₈- alkoxy-C₁-C₈-alkyl, C₁-C₁₀-alkylthio-C₁-C₆- alkyl, optionally halogen-, C₁-C₆-alkyl- or C₁-C₆-alkoxy-substituted C₃-C₈-cycloalkyl in which optionally one or two not directly adjacent ring members are replaced by oxygen and/or sulphur or represents in each case optionally halogen-, C₁-C₆-alkyl-, C₁-C₆- haloalkyl-, C₁-C₆-alkoxy-, C₁-C₆-haloalkoxy-, cyano- or nitro-substituted phenyl, phenyl-C₁-C₆-alkyl, or represents hydrogen if in the case of group (I-1) D is not hydrogen,
B represents hydrogen, C₁-C₁₂-alkyl or C₁-C₈- alkoxy-C₁-C₆-alkyl, or
A, B and the carbon atom to which they are attached represent saturated C₃-C₁₀-cycloalkyl or unsaturated C₅-C₁₀-cycloalkyl in which optionally one ring member is replaced by oxygen or sulphur and which is optionally mono- or disubstituted by C₁-C₈-alkyl, C₃-C₁₀- cycloalkyl, C₁-C₆-haloalkyl, C₁-C₈-alkoxy, C₁-C₈-alkylthio, halogen or phenyl, or
A, B and the carbon atom to which they are attached represent C₃-C₆-cycloalkyl which is substituted by an alkylenediyl group which optionally contains one or two not directly adjacent oxygen and/or sulphur atoms, or by an alkylenedioxyl or by an alkylenedithioyl group which, together with the carbon atom to which it is attached, forms a further five- to eight- membered ring, or
A, B and the carbon atom to which they are attached represent C₃-C₈-cycloalkyl or C₅-C₈- cycloalkenyl in which two substituents together with the carbon atoms to which they are attached represent in each case optionally C₁-C₆-alkyl-, C₁-C₆-alkoxy- or halogen- substituted C₂-C₆-alkanediyl, C₂-C₆-alkenediyl or C₄-C₆-alkanediendiyl in which optionally one methylene group is replaced by oxygen or sulphur,
D represents hydrogen, in each case optionally halogen-substituted C₁-C₁₂-alkyl, C₃-C₈- alkenyl, C₃-C₈-alkynyl, C₁-C₁₀-alkoxy-C₂-C₈- alkyl, poly-C₁-C₈-alkox_{Y}-C₂-C₈-alkyl, C₁-C₁₀- alkylthio-C₂-C₈-alkyl, optionally halogen-, C₁-C₄-alkyl-, C₁-C₄-alkoxy- or C₁-C₄-haloalkyl- substituted C₃-C₈-cycloalkyl in which optionally one ring member is replaced by oxygen or sulphur or represents optionally halogen-, C₁-C₆-alkyl-, C₁-C₆-haloalkyl-, C₁-C₆-alkoxy-, C₁-C₆-haloalkoxy-, cyano- or nitro-substituted phenyl,
A and D together represent in each case optionally substituted C₃-C₆-alkanediyl or C₃-C₆- alkenediyl in which optionally one methylene group is replaced by oxygen or sulphur, possible substituents being in each case: C₁-C₆-alkyl, C₁-C₆-alkoxy or a further C₃-C₆- alkanediyl grouping,
G represents chlorine, bromine or nitro.

2. Compounds of the formula (I) according to Claim 1 in which
Q represents oxygen, sulphur or the group N-D,
W represents hydrogen, chlorine, bromine or C₁-C₄-alkyl,
X represents fluorine, chlorine, bromine, C₁-C₄- alkyl, C₁-C₄-alkoxy, C₁-C₄-haloalkyl, C₁-C₄- haloalkoxy or cyano,
Y represents the radical 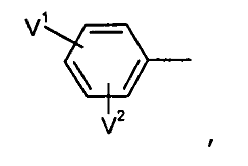
V¹ represents hydrogen, fluorine, chlorine, bromine, C₁-C₆-alkyl, C₁-C₄-alkoxy, C₁-C₂- haloalkyl, C₁-C₂-haloalkoxy, nitro or cyano, or represents phenyl which is optionally mono- or disubstituted by fluorine, chlorine, bromine, C₁-C₄-alkyl, C₁-C₄-alkoxy, C₁-C₂-haloalkyl, C₁-C₂-haloalkoxy, nitro or cyano,
V² represents hydrogen, fluorine, chlorine, bromine, C₁-C₄-alkyl, C₁-C₄-alkoxy, C₁-C₂- haloalkyl or C₁-C₂-haloalkoxy,
Z represents hydrogen, fluorine, chlorine, bromine, C₁-C₄-alkyl, C₁-C₂-haloalkyl, C₁-C₄- alkoxy or C₁-C₂-haloalkoxy,
A represents C₁-C₁₀-alkyl, C₁-C₈-alkoxy-C₁-C₆- alkyl, each of which is optionally mono- to pentasubstituted by fluorine or chlorine, represents C₃-C₇-cycloalkyl which is optionally mono- or disubstituted by fluorine, chlorine, C₁-C₄-alkyl or C₁-C₄-alkoxy and in which optionally one ring member is replaced by oxygen or sulphur, or represents phenyl or phenyl-C₁₂-C₄-alkyl, each of which is optionally mono- or disubstituted by fluorine, chlorine, bromine, C₁-C₄-alkyl, C₁-C₄-haloalkyl, C₁-C₄- alkoxy or C₁-C₄-haloalkoxy, or represents hydrogen if in the case of the group (I-1) D is not hydrogen,
B represents hydrogen or C₁-C₆-alkyl, or
A, B and the carbon atom to which they are attached represent saturated C₃-C₈-cycloalkyl in which optionally one ring member is replaced by oxygen or sulphur and which is optionally mono- or disubstituted by C₁-C₆-alkyl, C₅-C₆- cycloalkyl, C₁-C₃-haloalkyl, C₁-C₆-alkoxy, fluorine, chlorine or phenyl, with the proviso that D in group (I-1) represents hydrogen or C₁-C₄-alkyl,
D represents hydrogen, represents C₁-C₁₀-alkyl, C₃-C₆-alkenyl, C₁-C₄-alkoxy-C₂-C₄-alkyl or C₁-C₄-alkylthio-C₂-C₄-alkyl, each of which is optionally mono- to pentasubstituted by fluorine or chlorine, represents C₃-C₇- cycloalkyl which is optionally mono- or disubstituted by fluorine, chlorine, C₁-C₄- alkyl, C₁-C₄-alkoxy or C₁-C₂-haloalkyl and in which optionally one methylene group is replaced by oxygen or sulphur, or represents phenyl which is optionally mono- or disubstituted by fluorine, chlorine, bromine, C₁-C₄-alkyl, C₁-C₄-haloalkyl, C₁-C₄-alkoxy or C₁-C₄-haloalkoxy, or
A and D together represent optionally substituted C₃-C₅-alkanediyl in which one methylene group may be replaced by oxygen or sulphur, possible substituents being C₁-C₄-alkyl,
G represents chlorine, bromine or nitro.

3. Compounds of the formula (I) according to Claim 1, in which
Q represents oxygen, sulphur or the group N-D,
W represents hydrogen, chlorine, methyl or ethyl,
X represents chlorine, methyl, ethyl, n-propyl, isopropyl, methoxy, ethoxy, n-propoxy, isopropoxy, trifluoromethyl, difluoromethoxy, trifluoromethoxy or cyano,
Y represents the radical 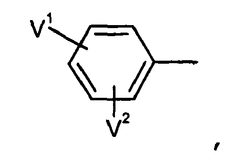
V¹ represents hydrogen, fluorine, chlorine, bromine, methyl, ethyl, n-propyl, isopropyl, n- butyl, isobutyl, tert-butyl, methoxy, ethoxy, n-propoxy, isopropoxy, trifluoromethyl, trifluoromethoxy, nitro or cyano,
V² represents hydrogen, fluorine, chlorine, methyl, ethyl, n-propyl, isopropyl, methoxy, ethoxy, trifluoromethyl or trifluoromethoxy,
Z represents hydrogen, fluorine, chlorine, methyl, ethyl, n-propyl or methoxy,
A represents C₁-C₆-alkyl or C₁-C₂-alkoxy-C₁-C₂- alkyl, C₃-C₆-cycloalkyl in which optionally one ring member is replaced by oxygen or sulphur or represents hydrogen if in the case of the group (I-1) D is not hydrogen,
B represents hydrogen, methyl, ethyl, or
A, B and the carbon atom to which they are attached represent saturated C₃-C₈-cycloalkyl in which optionally one ring member is replaced by oxygen or sulphur and which is optionally monosubstituted by methyl, ethyl, n-propyl, isopropyl, n-butyl, isobutyl, tert-butyl, trifluoromethyl, methoxy, ethoxy, n-propoxy, isopropoxy, n-butoxy or isobutoxy, with the proviso that D in group (I-1) represents hydrogen or C₁-C₃-alkyl,
D represents hydrogen, methyl, ethyl, n-propyl, isopropyl, cyclopropyl, cyclopentyl or cyclohexyl,
A, D together represent optionally substituted C₃-C₄-alkanediyl in which optionally one carbon atom is replaced by oxygen or sulphur,
G represents chlorine or nitro.

4. Compounds of the formula (I) according to Claim 1, in which
Q represents the group N-D,
W represents hydrogen, chlorine or methyl,
X represents chlorine, methyl or ethyl,
Y represents the radical 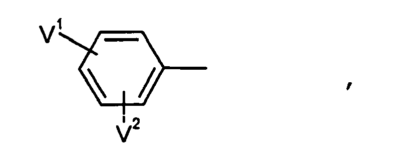
V¹ represents chlorine or methyl,
V² represents hydrogen or chlorine,
Z represents hydrogen or methyl,
A represents methyl, ethyl, propyl, isopropyl, n- butyl, isobutyl, s-butyl, tert-butyl, cyclopropyl, cyclopentyl or cyclohexyl,
B represents methyl,
A, B and the carbon atom to which they are attached represent saturated C₃-C₈-cycloalkyl in which optionally one ring member is replaced by oxygen or sulphur and which is optionally monosubstituted by methyl or, in the case of C₆- cycloalkyl, also by ethyl, n-propyl, isopropyl, n-butyl, isobutyl, tert-butyl, trifluoromethyl, methoxy, ethoxy, n-propoxy, isopropoxy, n- butoxy or isobutoxy,
D represents hydrogen,
G represents chlorine or nitro.

5. Compounds of the formula (I) according to Claim 1, in which
Q represents oxygen,
W represents hydrogen, chlorine or methyl,
X represents chlorine, methyl or ethyl,
Y represents the radical 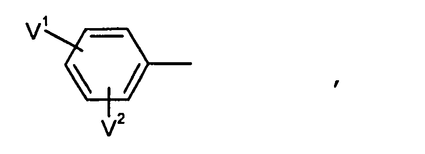
V¹ represents chlorine, fluorine, cyano or methyl,
V² represents hydrogen or chlorine,
Z represents hydrogen or methyl,
A represents methyl, ethyl, propyl, isopropyl, n- butyl, isobutyl, s-butyl, tert-butyl, cyclopropyl, cyclopentyl or cyclohexyl,
B represents methyl, or
A, B and the carbon atom to which they are attached represent saturated C₃-C₈-cycloalkyl in which optionally one ring member is replaced by oxygen or sulphur and which is optionally monosubstituted by methyl or, in the case of C₆-cycloalkyl, also by ethyl, n-propyl, isopropyl, n-butyl, isobutyl, tert-butyl, trifluoromethyl, methoxy, ethoxy, n-propoxy, isopropoxy, n-butoxy or isobutoxy,
G represents chlorine.

6. Compounds of the formula (I) according to Claim 1, in which
Q represents sulphur,
W represents hydrogen,
X represents chlorine or methyl,
Y represents the radical 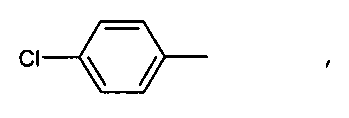
Z represents hydrogen or methyl,
A, B and the carbon atom to which they are attached represent saturated C₅-C₆-cycloalkyl in which optionally one ring member is replaced by oxygen and which is optionally substituted by methyl or methoxy,
G represents chlorine.

7. Process for preparing compounds of the formula (I) according to Claim 1, **characterized in that**, to obtain
A) compounds of the formulae (I-1) to (I-3) 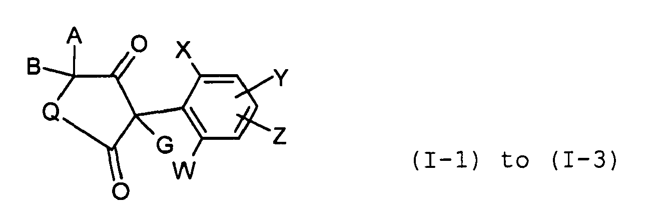 in which
A, B, Q, W, X, Y and Z are as defined above and
G represents halogen,
compounds of the formulae (II-1) to (II-3) 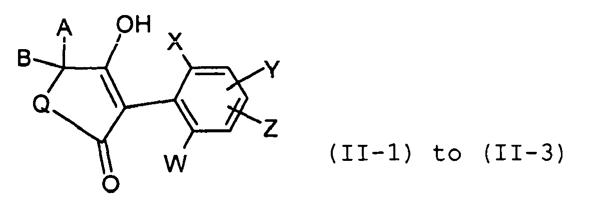 in which
A, B, Q, W, X, Y and Z are as defined above are reacted with halogenating agents in the presence of a solvent and, if appropriate, in the presence of a free-radical initiator, or
B) compounds of the formulae (I-1) to (I-3) 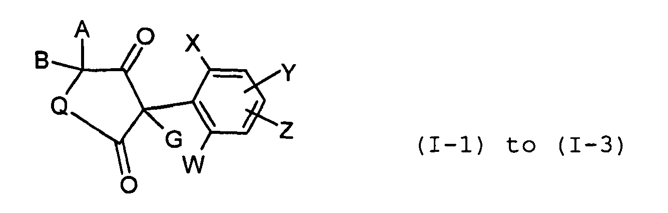 in which
A, B, Q, W, X, Y and Z are as defined above and
G represents nitro,
compounds of the formulae (II-1) to (II-3) 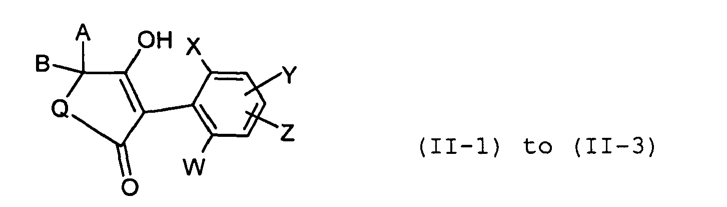 in which
A, B, Q, W, X, Y and Z are as defined above are reacted with nitrating agents in the presence of a solvent.

8. Pesticides and/or herbicides and/or fungicides, **characterized in that** they comprise at least one compound of the formula (I) according to Claim 1.

9. Compounds of the formula (I) according to Claim 1 for use in a method for controlling animal pests and/or unwanted vegetation and/or fungi, **characterized in that** compounds of the formula (I) according to Claim 1 are allowed to act on pests and/or their habitat.

10. Compounds of the formula (I) according to Claim 1 for use for controlling animal pests and/or unwanted vegetation and/or fungi.

11. Process for preparing pesticides and/or herbicides and/or fungicides, **characterized in that** compounds of the formula (I) according to Claim 1 are mixed with extenders and/or surfactants.

12. Use of compounds of the formula (I) according to Claim 1 for preparing pesticides and/or herbicides and/or fungicides.

## Revendications

1. Composés de formule (I), 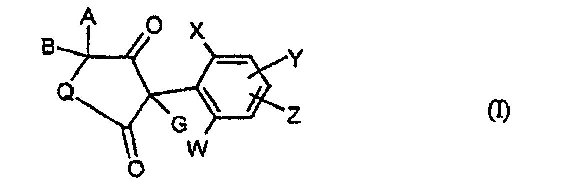 où
Q représente oxygène, soufre ou le groupe N-D,
W représente hydrogène, halogène, C₁-C₆-alkyle ou C₁- C₆-alcoxy,
X représente halogène, C₁-C₆-alkyle, C₁-C₆- halogénoalkyle, C₁-C₆-alcoxy, C₁-C₆-halogénoalcoxy, cyano ; ou phényle le cas échéant substitué par halogène, C₁-C₆-alkyle, C₁-C₆-alcoxy, C₁-C₉- halogénoalkyle, C₁-C₄-halogénoalcoxy, nitro ou cyano,
Y représente un des radicaux 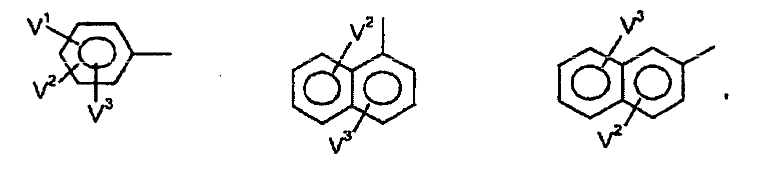 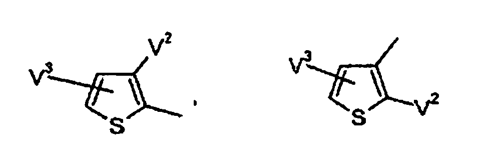
V¹ représente hydrogène, halogène, C₁-C₁₂-alkyle, C₁- C₆-alcoxy, C₁-C₆-alkylthio, C₁-C₆-alkylsulfinyle, C₁-C₆-alkylsulfonyle, C₁-C₄-halogénoalkyle, C₁-C₄- halogénoalcoxy, nitro, cyano ; ou phényle le cas échéant monosubstitué à disubstitué par halogène, C₁-C₆-alkyle, C₁-C₆-alcoxy, C₁-C₄-halogénoalkyle, C₁-C₉-halogénoalcoxy, nitro ou cyano,
V² et V³ représentent, indépendamment l'un de l'autre, hydrogène, halogène, C₁-C₆-alkyle, C₁-C₆-alcoxy, C₁- C₄-halogénoalkyle ou C₁-C₄-halogénoalcoxy,
Z représente hydrogène, halogène, C₁-C₆-alkyle, C₁- C₆-halogénoalkyle, C₁-C₆-alcoxy, C₁-C₆- halogénoalcoxy, nitro ou cyano,
A représente C₁-C₁₂-alkyle, C₃-C₈-alcényle, C₁-C₁₀- alcoxy-C₁-C₈-alkyle, poly-C₁-C₈-alcoxy-C₁-C₈-alkyle, C₁-C₁₀-alkylthio-C₁-C₆-alkyle à chaque fois le cas échéant substitué par halogène, C₃-C₈-cycloalkyle le cas échéant substitués par halogène; C₁-C₆- alkyle ou C₁-C₆-alcoxy, dans lequel le cas échéant un ou deux éléments de cycle non directement adjacents sont remplacés par oxygène et/ou soufre ; ou phényle, phényl-C₁-C₆-alkyle à chaque fois le cas échéant substitués par halogène, C₁-C₆- alkyle, C₁-C₆-halogénoalkyle, C₁-C₆-alcoxy, C₁-C₆- halogénoalcoxy, cyano ou nitro ; ou hydrogène, lorsque, dans le cas du groupe (I-1), D est différent d'hydrogène,
B représente hydrogène, C₁-C₁₂-alkyle ou C₁-C₈-alcoxy- C₁-C₆-alkyle ou
A, B et l'atome de carbone auquel ils sont liés représentent C₃-C₁₀-cycloalkyle saturé ou C₅-C₁₀- cycloalkyle insaturé, dans lesquels le cas échéant un élément de cycle est remplacé par oxygène ou soufre et qui sont le cas échéant monosubstitués ou disubstitués par C₁-C₈-alkyle, C₃-C₁₀- cycloalkyle, C₁-C₆-halogénoalkyle, C₁-C₈-alcoxy, C₁- C₈-alkylthio, halogène ou phényle ou
A, B et l'atome de carbone auquel ils sont liés représentent C₃-C₆-cycloalkyle, qui est substitué
A, B par un groupe alkylènediyle contenant le cas échéant un ou deux atomes d'oxygène et/ou de soufre non directement adjacents ou par un groupe alkylènedioxyle ou par un groupe alkylènedithioyle, qui forme avec l'atome de carbone auquel il est lié un autre cycle de cinq à huit chaînons ou et l'atome de carbone auquel ils sont liés représentent C₃-C₈-cycloalkyle ou C₅-C₈- cycloalcényle, dans lesquels deux substituants, ensemble avec les atomes de carbone auxquels ils sont liés, représentent C₂-C₆-alcanediyle, C₂-C₆- alcènediyle ou C₄-C₆-alcanediènediyle à chaque fois le cas échéant substitués par Cₗ-C₆-alkyle, C₁-C₆- alcoxy ou halogène, dans lesquels le cas échéant un groupe méthylène est remplacé par oxygène ou soufre,
D représente hydrogène; C₁-C₁₂-alkyle, C₃-C₈- alcényle, C₃-C₈-alcynyle, C₁-C₁₀-alcoxy-C₂-C₈- alkyle, poly-C₁-C₈-alcoxy-C₂-C₈-alkyle, C₁-C₁₀- alkylthio-C₂-C₈-alkyle à chaque fois le cas échéant substitué par halogène, C₃-C₈-cycloalkyle le cas échéant substitué par halogène, C₁-C₄-alkyle, C₁- C₄-alcoxy ou C₁-C₄-halogénoalkyle, dans lequel le cas échéant un élément de cycle est remplacé par oxygène ou soufre ; ou phényle le cas échéant substitué par halogène, C₁-C₆-alkyle, C₁-C₆- halogénoalkyle, C₁-C₆-alcoxy, C₁-C₆-halogénoalcoxy, cyano ou nitro,
A et D représentent ensemble C₃-C₆-alcanediyle ou C₃- C₆-alcènediyle à chaque fois le cas échéant substitués, dans lesquels le cas échéant un groupe méthylène est remplacé par oxygène ou soufre et où les substituants qui entrent à chaque fois en considération sont : C₁-C₆-alkyle, C₁-C₆-alcoxy ou un autre groupement C₃-C₆-alcanediyle,
G représente chlore, brome ou nitro.

2. Composés de formule (I) selon la revendication 1, où
Q représente oxygène, soufre ou le groupe N-D,
W représente hydrogène, chlore, brome ou C₁-C₄- alkyle,
X représente fluor, chlore, brome, C₁-C₄-alkyle, C₁- C₄-alcoxy, C₁-C₄-halogénoalkyle, C₁-C₄- halogénoalcoxy ou cyano,
Y représente le radical 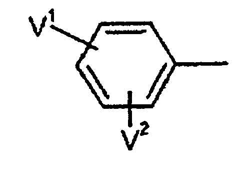
V¹ représente hydrogène, fluor, chlore, brome, C₁-C₆- alkyle, C₁-C₄-alcoxy, C₁-C₂-halogénoalkyle, C₁-C₂- halogénoalcoxy, nitro ou cyano ; phényle le cas échéant monosubstitué ou disubstitué par fluor, chlore, brome, C₁-C₄-alkyle, C₁-C₄-alcoxy, C₁-C₂- halogénoalkyle, C₁-C₂-halogénoalcoxy, nitro ou cyano,
V² représente hydrogène, fluor, chlore, brome, C₁-C₄- alkyle, C₁-C₄-alcoxy, C₁-C₂-halogénoalkyle ou C₁-C₂- halogénoalcoxy,
Z représente hydrogène, fluor, chlore, brome, C₁-C₄- alkyle, C₁-C₂-halogénoalkyle, C₁-C₄-alcoxy ou C₁-C₂- halogénoalcoxy,
A représente C₁-C₁₀-alkyle, C₁-C₈-alcoxy-C₁-C₆-alkyle à chaque fois le cas échéant monosubstitués à pentasubstitués par fluor ou chlore: C₃-C₇- cycloalkyle le cas échéant monosubstitué à disubstitué par fluor, chlore, C₁-C₁-alkyle ou C₁- C₄-alcoxy, dans lequel le cas échéant un élément de cycle est remplacé par oxygène ou soufre ; ou phényle ou phényl-C₁-C₄-alkyle à chaque fois le cas échéant monosubstitué à disubstitué par fluor, chlore, brome, C₁-C₄-alkyle, C₁-C₄-halogénoalkyle, C₁-C₄-alcoxy ou C₁-C₄-halogénoalcoxy; ou
B hydrogène, lorsque, dans le cas du groupe (I-1) D est différent d'hydrogène, représente hydrogène ou C₁-C₆-alkyle, ou
A, B et l'atome de carbone auquel ils sont liés représentent C₃-C₈-cycloalkyle saturé, dans lequel le cas échéant un élément de cycle est remplacé par oxygène ou soufre et qui est le cas échéant monosubstitué à disubstitué par C₁-C₆-alkyle, C₅- C₆-cycloalkyle, C₁-C₃-halogénoalkyle, C₁-C₆-alcoxy, fluor, chlore ou phényle, à condition que D dans le groupe (I-1) représente hydrogène ou C₁-C₄- alkyle,
D représente hydrogène; C₁-C₁₀-alkyle, C₃-C₆- alcényle, C₁-C₄-alcoxy-C₂-C₄-alkyle ou C₁-C₄- alkylthio-C₂-C₄-alkyle à chaque fois le cas échéant monosubstitués à pentasubstitués par fluor ou chlore ; C₃-C₇-cycloalkyle le cas échéant monosubstitué à disubstitué par fluor, chlore, C₁- C₄-alkyle, C₁-C₄-alcoxy ou C₁-C₂-halogénoalkyle, dans lequel le cas échéant un groupe méthylène est remplacé par oxygène ou soufre ; ou phényle le cas échéant monosubstitué ou disubstitué par fluor, chlore, brome, C₁-C₄-alkyle, C₁-C₄-halogénoalkyle, C₁-C₄-alcoxy ou C₁-C₄-halogénoalcoxy, ou
A et D représentent ensemble C₃-C₅-alcanediyle le cas échéant substitué, dans lequel un groupe méthylène peut être remplacé par oxygène ou soufre et où les substituants qui entrent en considération sont C₁- C₄-alkyle,
G représente chlore, brome ou nitro.

3. Composé de formule (I) selon la revendication 1, où
Q représente oxygène, soufre ou le groupe N-D,
W représente hydrogène, chlore, méthyle ou éthyle,
X représente chlore, méthyle, éthyle, n-propyle, iso-propyle, méthoxy, éthoxy, n-propoxy, iso- propoxy, trifluorométhyle, difluorométhoxy, trifluorométhoxy ou cyano,
Y représente le radical 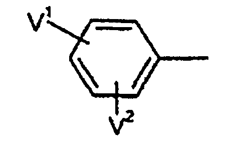
V¹ représente hydrogène, fluor, chlore, brome, méthyle, éthyle, n-propyle, iso-propyle, n-butyle, iso-butyle, tert-butyle, méthoxy, éthoxy, n- propoxy, iso-propoxy, trifluorométhyle, trifluorométhoxy, nitro ou cyano,
V² représente hydrogène, fluor, chlore, méthyle, éthyle, n-propyle, iso-propyle, méthoxy, éthoxy, trifluorométhyle ou trifluorométhoxy,
Z représente hydrogène, fluor, chlore, méthyle, éthyle, n-propyle ou méthoxy,
A représente C₁-C₆-alkyle ou C₁-C₂-alcoxy-C₁-C₂- alkyle, C₃-C₆-cycloalkyle, dans lequel le cas échéant un élément de cycle est remplacé par oxygène ou soufre ; ou hydrogène, lorsque dans le cas du groupe (I-1) D est différent d'hydrogène,
B représente hydrogène, méthyle ou éthyle ou
A, B et l'atome de carbone auquel ils sont liés représentent C₃-C₈-cycloalkyle saturé, dans lequel le cas échéant un élément de cycle est remplacé par oxygène ou soufre et qui est le cas échéant monosubstitué par méthyle, éthyle, n-propyle, isopropyle, n-butyle isobutyle, tert-butyle, trifluorométhyle, méthoxy, éthoxy, n-propoxy, iso- propoxy, n-butoxy ou iso-butoxy, à condition que D dans le groupe (I-1) représente hydrogène ou C₁-C₃- alkyle,
D représente hydrogène, méthyle, éthyle, n-propyle, isopropyle, cyclopropyle, cyclopentyle ou cyclohexyle,
A, D représentent ensemble C₃-C₄-alcanediyle le cas échéant substitué, dans lequel le cas échéant un atome de carbone est remplacé par oxygène ou soufre,
G représente chlore ou nitro.

4. Composés de formule (I) selon la revendication 1, où
Q représente le groupe N-D,
W représente hydrogène, chlore ou méthyle,
X représente chlore, méthyle ou éthyle,
Y représente le radical 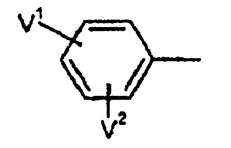
V¹ représente chlore ou méthyle,
V² représente hydrogène ou chlore,
Z représente hydrogène ou méthyle,
A représente méthyle, éthyle, propyle, isopropyle, n-butyle, isobutyle, s-butyle, tert-butyle, cyclopropyle, cyclopentyle ou cyclohexyle,
B représente méthyle,
A, B et l'atome de carbone auquel ils sont liés représentent C₃-C₈-cycloalkyle saturé, dans lequel le cas échéant un élément de cycle est remplacé par oxygène ou soufre et qui est le cas échéant monosubstitué par méthyle ou, dans le cas de C₆- cycloalkyle, également par éthyle, n-propyle, isopropyle, n-butyle, isobutyle, tert-butyle, trifluorométhyle, méthoxy, éthoxy, n-propoxy, iso- propoxy, n-butoxy ou iso-butoxy,
D représente hydrogène,
G représente chlore ou nitro.

5. Composés de formule (I) selon la revendication 1, où
Q représente oxygène,
W représente hydrogène, chlore ou méthyle,
X représente chlore, méthyle ou éthyle,
Y représente le radical 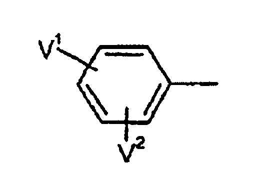
V¹ représente chlore, fluor, cyano ou méthyle,
V² représente hydrogène ou chlore,
Z représente hydrogène ou méthyle,
A représente méthyle, éthyle, propyle, isopropyle, n-butyle, isobutyle, s-butyle, tert-butyle, cyclopropyle, cyclopentyle ou cyclohexyle,
B représente méthyle ou
A, B et l'atome de carbone auquel ils sont liés représentent C₃-C₈-cycloalkyle saturé, dans lequel le cas échéant un élément de cycle est remplacé par oxygène ou soufre et qui est le cas échéant monosubstitué par méthyle ou, dans le cas de C₆- cycloalkyle, également par éthyle, n-propyle, isopropyle, n-butyle, isobutyle, tert-butyle, trifluorométhyle, méthoxy, éthoxy, n-propoxy, iso- propoxy, n-butoxy ou iso-butoxy,
G représente chlore.

6. Composés de formule (I) selon la revendication 1, où
Q représente soufre,
W représente hydrogène,
X représente chlore ou méthyle,
Y représente le radical 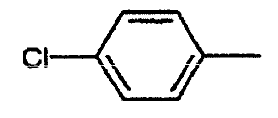
Z représente hydrogène ou méthyle,
A, B et l'atome de carbone auquel ils sont liés représentent C₅-C₆-cycloalkyle saturé, dans lequel le cas échéant un élément de cycle est remplacé par oxygène et qui est le cas échéant substitué par méthyle ou méthoxy,
G représente chlore.

7. Procédé pour la préparation de composés de formule (I) selon la revendication 1, **caractérisé en ce que**, pour obtenir
A) des composés des formules (I-1) à (I-3) 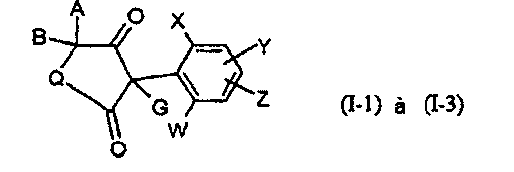 où
A, B, Q, W, X, Y et Z, présentent la signification susmentionnée et
G représente halogène,
on transforme des composés des formules (II-1) à (II-3) 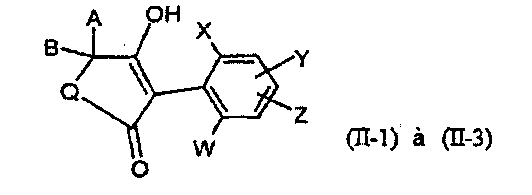 où
A, B, Q, W, X, Y et Z ont la signification indiquée ci-dessus avec un agent d'halogénation en présence d'un solvant et le cas échéant en présence d'un initiateur de radicaux ou
B) des composés des formules (I-1) à (I-3) 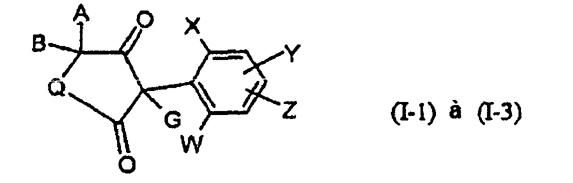 où
A, B, Q, W, X, Y et Z présentent la signification susmentionnée et
G représente nitro,
on transforme des composés des formules (II-1) à
(II-3) 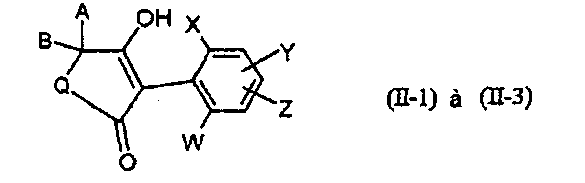 où
A, B, Q, W, X, Y et Z ont la signification indiquée ci-dessus
avec des réactifs de nitruration en présence d'un solvant.

8. Agents de lutte contre des organismes nuisibles et/ou herbicides et/ou fongicides, **caractérisés par** une teneur en au moins un composé de formule (I) selon la revendication 1.

9. Composés de formule (I) selon la revendication 1 destinés à une utilisation dans des procédés pour lutter contre des organismes nuisibles animaux et/ou une couverture végétale non souhaitée et/ou des champignons non souhaités, **caractérisés en ce qu'**on laisse agir des composés de formule (I) selon la revendication 1 sur des organismes nuisibles et/ou leur espace de vie.

10. Composés de formule (I) selon la revendication 1 destinés à une utilisation pour lutter contre des organismes nuisibles animaux et/ou une couverture végétale non souhaitée et/ou des champignons non souhaités.

11. Procédé pour la préparation d'agents de lutte contre les organismes nuisibles et/ou des herbicides et/ou des fongicides, **caractérisé en ce qu'**on mélange des composés de formule (I) selon la revendication 1 avec des agents d'allongement et/ou des substances tensioactives.

12. Utilisation de composés de formule (I) selon la revendication 1 pour la préparation d'agents de lutte contre les organismes nuisibles et/ou des herbicides et/ou des fongicides.
